(19) **Europäisches Patentamt**
**European Patent Office**
**Office européen des brevets**

(11) **EP 1 820 024 B1**

(12) **EUROPEAN PATENT SPECIFICATION**

(45) Date of publication and mention
of the grant of the patent:
**13.06.2012 Bulletin 2012/24**

(51) Int Cl.:
*G01N 33/58* (2006.01)   *G01N 33/53* (2006.01)
*G01N 21/64* (2006.01)

(21) Application number: **05852896.9**

(22) Date of filing: **01.12.2005**

(86) International application number:
**PCT/US2005/043812**

(87) International publication number:
**WO 2006/060739 (08.06.2006 Gazette 2006/23)**

(54) **UBIQUITIN LIGASE ASSAYS AND RELATED REAGENTS**

UBIQUITIN-LIGASE-ASSAYS UND VERWANDTE REAGENZIEN

ESSAIS PORTANT SUR L'UBIQUITINE LIGASE ET REACTIFS ASSOCIES

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **01.12.2004 US 632317 P
01.12.2004 US 632048 P
30.12.2004 US 640710 P
07.02.2005 US 650944 P**

(43) Date of publication of application:
**22.08.2007 Bulletin 2007/34**

(60) Divisional application:
**11001146.7**

(73) Proprietor: **Proteologics, Ltd**
**76124 Rehovot (IL)**

(72) Inventors:
• **TUVIA, Shmuel**
**42490 Netanya (IL)**
• **REISS, Yuval**
**Kiriat-ono (IL)**

(74) Representative: **Vossius & Partner**
**Siebertstrasse 4**
**81675 München (DE)**

(56) References cited:
**WO-A-00/50896      WO-A-01/75145
WO-A-02/066982     WO-A-2004/099388
US-A1- 2002 197 606**

• **MOHAMMED SARWAR NASIR ET AL:**
**"Fluorescence Polarization: an analytical tool for immunoassay and drug discovery"** COMBINATORIAL CHEMISTRY AND HIGH THROUGHPUT SCREENING, HILVERSUM, NL, vol. 2, no. 4, 1999, pages 177-190, XP001062258 ISSN: 1386-2073
• **LUNDBLAD J R ET AL:** "Fluorescence polarization analysis of protein-DNA and protein-protein interactions." MOLECULAR ENDOCRINOLOGY (BALTIMORE, MD.) JUN 1996, vol. 10, no. 6, June 1996 (1996-06), pages 607-612, XP002377268 ISSN: 0888-8809
• **TIRAT ET AL:** "Synthesis and characterization of fluorescent ubiquitin derivatives as highly sensitive substrates for the deubiquitinating enzymes UCH-L3 and USP-2" ANALYTICAL BIOCHEMISTRY, ACADEMIC PRESS, NEW YORK, NY, US, vol. 343, no. 2, 15 August 2005 (2005-08-15), pages 244-255, XP005001573 ISSN: 0003-2697
• **SUN LIJUN ET AL:** "The novel functions of ubiquitination in signaling." CURRENT OPINION IN CELL BIOLOGY. APR 2004, vol. 16, no. 2, April 2004 (2004-04), pages 119-126, XP002377294 ISSN: 0955-0674
• **SHCHERBIK NATALIA ET AL:** "Ub on the move." JOURNAL OF CELLULAR BIOCHEMISTRY. 1 SEP 2004, vol. 93, no. 1, 1 September 2004 (2004-09-01), pages 11-19, XP002377295 ISSN: 0730-2312

EP 1 820 024 B1

**Description**

BACKGROUND

The ubiquitin-mediated proteolysis system is the major pathway for the selective, controlled degradation of intracellular proteins in eukaryotic cells. Ubiquitin modification of a variety of protein targets within the cell is important in a number of basic cellular functions such as regulation of gene expression, regulation of the cell-cycle, modification of cell surface receptors, biogenesis of ribosomes, and DNA repair, and therefore, the ubiquitin system has been implicated in the pathogenesis of numerous disease states, including oncogenesis, inflammation, viral infection, CNS disorders, and metabolic dysfunction.

One major function of the ubiquitin-mediated system is to control the half lives of cellular proteins. The half-life of different proteins can range from a few minutes to several days and can vary considerably depending on the cell-type, nutritional and environmental conditions, as well as the stage of the cell-cycle. Targeted proteins undergoing selective degradation, presumably through the actions of a ubiquitin-dependent proteosome, are covalently tagged with ubiquitin through the formation of an isopeptide bond between the C-terminal glycyl residue of ubiquitin and a specific lysyl residue in the substrate protein. This process is catalyzed by a ubiquitin-activating enzyme (E1) and a ubiquitin-conjugating enzyme (E2), and may also require auxiliary substrate recognition proteins (E3s). Following the linkage of the first ubiquitin chain, additional molecules of ubiquitin may be attached to lysine side chains of the previously conjugated moiety to form multi-ubiquitin chains.

The conjugation of ubiquitin to protein substrates is a multi-step process. In an initial ATP-dependent step, a thioester is formed between the C-terminus of ubiquitin and an internal cysteine residue of an E1 enzyme. Activated ubiquitin is then transferred to a specific cysteine on one of several E2 enzymes. Finally, these E2 enzymes donate ubiquitin to protein substrates. Substrates are recognized either directly by ubiquitin-conjugated enzymes or by associated substrate recognition proteins, the E3 proteins, also known as ubiquitin ligases.

In addition to the 76-amino acid ubiquitin, there is a family of ubiquitin-like protein modifiers that are low molecular weight polypeptides (76-165 amino acids) and share between 10% and 55% sequence identity to ubiquitin. See, e.g., Wong et al., Drug Discovery in the Ubiquitin Regulatory Pathway, DDT 8(16): 746-54, August 2003; Schwartz & Hochstrasser, A Superfamily of Protein Tags: Ubiquitin, SUMO and Related Modifiers, Trends Biochem. Sci. 28(6): 321-28, June 2003. Although ubiquitin and each ubiquitin-like protein modifier direct distinct sets of biological consequences and each requires distinct conjugation and deconjugation machinery, they share a similar cascade mechanism involving an activating enzyme (E1), a conjugating enzyme (E2), and perhaps an auxiliary substrate recognition protein (E3, also termed ligase).

Genome mining efforts have identified at least 530 human genes that encode enzymes responsible for conjugation and deconjugation of ubiquitin or ubiquitin-like protein modifiers. See, e.g., Wong et al., supra. A multitude of E3s reflect their roles as specificity determinants; as a modular system, each E2-E3 pair appears to recognize a distinct set of cellular substrates. For example, the same E2 in conjunction with different E3s may recognize distinct substrates. The human genome encodes 391 potential E3s, as defined by the presence of HECT, RING finger, PHD or U-box domains. Wong et al., supra. The domains mediate the interaction of the E3 with the E2. E3s encompass a broad spectrum of molecular architectures ranging from large multimeric complexes (e.g., anaphase promoting complex or APC), in which E2 binding, substrate recognition, and regulatory functions reside in separate subunits, to relatively simple single component enzyme (e.g., murine double minute or MDM2) in which all necessary functions are incorporated into one polypeptide.

Detection of E3 autoubiquitination has been performed using homogeneous time-resolved fluorescence (HTRF) platforms. For example, Tularik, Inc. developed a high throughput time-resolved fluorescence resonance energy transfer assay for TRAF6 ubiquitin polymerization. (Hong, C. A. et al. Assay Drug Dev Technol. 2003 Feb;1 1(1 Pt 2):175-80.) Similarly, Roche developed an assay for P53 ubiquitination using the same technique. (Yabuki, N. et al. Comb Chem High Throughput Screen 1999 Oct;2(5):279-87.). Additionally, an ELISA method for an E3 autoubiquitination assay was described by Rigel Pharmaceuticals (US Pat. Nos. 6,740,495 and 6,737,244).

SUMMARY

As important regulatory mechanisms underlying diverse biological pathways, ubiquitin and ubiquitin-like protein modification systems present novel targets in the treatment of diseases. Accordingly, it is an object of the disclosure to provide assay systems and reagents for measuring the attachment of ubiquitin or other ubiquitin-like modifiers to various proteins, particularly E3 proteins.

The disclosure provides, in part, methods and reagents for evaluating the attachment of ubiquitin or a ubiquitin-like protein to a second protein, resulting in a decrease in the rotational freedom of the ubiquitin or ubiquitin-like polypeptide as characterised by the appended claims. One or more physical characteristics of the ubiquitin or ubiquitin-like protein may be measured in order to detect changes in rotational freedom, thereby providing a measure of the attachment of ubiquitin to a second protein. As an example, light emitted by a fluorescently labeled ubiquitin protein will be relatively anisotropic (non-polarized) when the ubiquitin has a high degree of rotational freedom. As the rotational freedom decreases, which occurs when the ubiquitin is conjugated to a second protein, the emitted light becomes increasingly isotropic (polarized). Thus, by detecting the polarization of light emitted by a fluorescently labeled ubiquitin protein ("fluorescence polarization", or "FP"), it is possible to assess the degree to which the labeled ubiquitin has become conjugated to other proteins. This principle may be applied regardless of whether the ubiquitin is attached to a different second protein or whether the ubiquitin forms a chain of one or more ubiquitin molecules. The same principle will also apply for ubiquitin-like polypeptides as well as polypeptides that bind polyubiquitin chains. Ubiquitin or ubiquitin-like polypeptides may be labeled directly (e.g., by conjugation to a fluorophore or by expression as a fusion with a fluorescent protein) or indirectly (e.g., by binding to a labeled antibody or other labeled binding protein). Additionally, labeling may be performed before or after any enzymatic reaction. For example, a reaction designed to generate ubiquitin-target protein conjugates may be performed with unlabeled ubiquitin, and the degree of conjugation may be detected afterwards by adding a fluorescently labeled anti-Ub antibody and detecting the polarization of light emitted from the Ub-antibody complex. In a preferred embodiment, a conjugation reaction is carried out using a ratio of labeled:unlabeled ubiquitin or ubiquitin-like polypeptide, and changes in rotational freedom are measured as a time course during the conjugation reaction.

Also described herein are methods and reagents for evaluating the attachment of a recognition element to a second protein, resulting in a decrease in the rotational freedom of the recognition element. As described herein, the term "recognition element" refers to a polypeptide that can bind a polyubiquitin chain (dimer or greater) or be used to assay for polyubiquitin formation on a target protein. For example, proteins such as S5a, TAB2, and TAB3 bind polyubiquitin. They can be labeled directly or indirectly with a fluorophore, such as XL665. Light emitted by a fluorescently labeled recognition element, such as a fluorescently labeled S5a protein, will be relatively anisotropic (non-polarized) when the recognition element has a high degree of rotational freedom. As the rotational freedom decreases, which occurs when the recognition element (e.g., S5a) is conjugated to a second polypeptide, such as a polyubiquitin chain, the emitted light becomes increasingly isotropic (polarized). Thus, by detecting the polarization of light emitted by a fluorescently labeled recognition element, it is possible to assess the degree to which the labeled recognition element has become conjugated to other proteins, such as polyubiquitinated proteins.

Further described herein are methods for assaying E3 ubiquitination activity by using measures of rotational freedom to assess the conjugation of ubiquitin to form poly-ubiquitin chains or to form complexes with a target protein. A target protein may be poly- or mono-ubiquitinated. In those instances where conjugation of ubiquitin is dependent on E3, the E3 activity may be accurately measured through measurement of formation of poly-ubiquitin chains or conjugation of a ubiquitin to an E3 of interest (e.g., autoubiquitination) or conjugation of a ubiquitin to an E3 substrate protein of interest. Participation of other proteins in ubiquitin conjugation may be evaluated similarly.

The assay may employ fluorescently labeled ubiquitin. For example, a fluorescently labeled ubiquitin and other components of the ubiquitin system are used to create a signal. Also described herein is a method that employs fluorescently labeled ubiquitin and unlabeled ubiquitin. The labeled and unlabeled ubiquitin are initially incubated in conditions that facilitate the formation of ubiquitin conjugates (e.g., with an E3 of interest, and optionally with an E2 and an E1). As the labeled and unlabeled ubiquitin are incorporated randomly into growing poly-ubiquitin chains, hybrid chains that contain fluorescently labeled ubiquitin are synthesized and are subsequently measured by fluorescence polarization. Further described herein are methods employing fluorescently labeled ubiquitin to detect monoubiquitinated E3 polypeptides. For example; labeled ubiquitin is initially incubated with an E3 of interest (and optionally an E2 or an E2 and an E1). As a labeled ubiquitin polypeptide is attached to the E3, hybrid E3/labeled ubiquitin is synthesized and can subsequently be detected by FP. A high fluorescence polarization signal will occur when a labeled ubiquitin polypeptide becomes bound to a high molecular weight E3 or is incorporated into a ubiquitin polychain (dimer or greater). If E3 conjugation ability is interrupted (by e.g., a small molecule or antibody) the emission of the fluorescently labeled ubiquitin is depolarized, leading to a low fluorescence polarization signal.

In certain aspects, the disclosure provides methods for evaluating the conjugation of a ubiquitin or ubiquitin-like polypeptide to a second polypeptide as characterised by the appended claims. The method comprises evaluating the rotational freedom of ubiquitin polypeptide in a mixture, wherein a decrease in rotational freedom of the ubiquitin polypeptide relative to unconjugated ubiquitin polypeptide indicates that the ubiquitin polypeptide is conjugated to a second polypeptide as characterised by the appended claims. The ubiquitin may be labeled directly (e.g., with a fluorophore) or indirectly. The rotational freedom of the labeled ubiquitin may be detected by detecting fluorescence polarization. Evaluating the rotational freedom of ubiquitin polypeptide in a mixture may comprise: a) providing a mixture comprising an E3 ubiquitin ligase, and a ubiquitin polypeptide; and b) detecting the rotational freedom of the ubiquitin polypeptide. The mixture may further comprise a substrate of the E3 ubiquitin ligase and may also comprise an E1 and an E2. Similar methods are provided for use with ubiquitin-like polypeptides as characterised by the appended claims.

Also described herein are methods of detecting the attachment of ubiquitin or ubiquitin-like protein to a target protein. A method may comprise: detecting, in a mixture comprising the target protein and a fluorescently labeled ubiquitin, the attachment of the labeled ubiquitin to the target protein by detecting fluorescence polarization. The mixture may further comprise an E3 polypeptide, and an E2 polypeptide and/or an E1 polypeptide. The ubiquitin may be directly labeled with a fluorophore such as fluorescein or rhodamine. Optionally, the target protein is an E3 protein such as a POSH protein, a cbl-b protein or a PEM-3-like protein, or a portion thereof that is ubiquitinated. The attachment of more than one ubiquitin to the target protein may be detected, and the target protein may be ubiquitin itself (in which case the method detects multimerization of ubiquitin). The mixture may include both labeled and unlabeled ubiquitin. Similar methods are provided for use with ubiquitin-like polypeptides.

In certain aspects, the disclosure provides methods for identifying a test compound that modulates ubiquitination (or conjugation to a ubiquitin-like protein) of a target protein, comprising a) providing a mixture comprising the target protein and a fluorescently labeled ubiquitin; and b) detecting the attachment of labeled ubiquitin to the protein by fluorescence polarization in the presence and absence of the test compound, wherein if the fluorescence polarization of the labeled ubiquitin in the presence of the compound is altered relative to the fluorescence polarization of the labeled ubiquitin in the absence of the test compound, a test compound that modulates ubiquitination of the protein is identified. Thus a decrease in fluorescence polarization may indicate that the test compound inhibits ubiquitin conjugation, while an increase in fluorescence polarization may indicate that the test compound promotes ubiquitin conjugation. In certain aspects, the ubiquitin is directly labeled with a fluorophore. The target protein may be an E3, such as a POSH protein, a cbl-b protein or a PEM-3-like protein, or a portion thereof that is ubiquitinated. The attachment of more than one ubiquitin to the target protein may be detected, and the target protein may be ubiquitin itself (in which case the method detects multimerization of ubiquitin). The mixture may include both labeled and unlabeled ubiquitin. The mixture may comprise an E2 and/or an E1. Similar methods are provided for use with ubiquitin-like polypeptides as characterised by the appended claims.

In certain aspects, the disclosure provides methods of evaluating the conjugation of a ubiquitin-like polypeptide to a second polypeptide as characterised by the appended claims. The method comprises evaluating the rotational freedom of ubiquitin-like polypeptide in a mixture, wherein a decrease in rotational freedom of the ubiquitin-like polypeptide relative to unconjugated ubiquitin-like polypeptide indicates that the ubiquitin-like polypeptide is conjugated to a second polypeptide. The ubiquitin-like polypeptide may be labeled directly (e.g., with a fluorophore) or indirectly. Rotational freedom of the labeled ubiquitin-like polypeptide may be detected by detecting fluorescence polarization. Evaluating the rotational freedom of ubiquitin-like polypeptide in a mixture may comprises: a) providing a mixture comprising a ligase (a protein that mediates attachment of the ubiquitin-like polypeptide to a substrate protein), and a ubiquitin-like polypeptide; and b) detecting the rotational freedom of the ubiquitin-like polypeptide. The mixture may further comprise a substrate of the ligase. The ubiquitin-like polypeptide is selected from the group consisting of: NEDD8, ISG15, SUMO1, SUM02, SUM03, APG12, and APG8.

Also described herein are methods of detecting the attachment of ubiquitin-like polypeptide to a target protein. A method may comprise: detecting, in a mixture comprising the target protein and a fluorescently labeled ubiquitin-like polypeptide, the attachment of the labeled ubiquitin-like polypeptide to the target protein by detecting fluorescence polarization. The mixture may further comprise a ligase polypeptide. The ubiquitin-like polypeptide may be directly labeled with a fluorophore. The attachment of more than one ubiquitin-like polypeptide to the target protein may be detected, and the target protein may itself be a ubiquitin-like polypeptide. The ubiquitin-like polypeptide may be selected from the group consisting of: NEDD8, ISG15, SUMO1, SUMO2, SUM03, APG12, and APG8. The mixture may further comprise unlabeled ubiquitin-like polypeptide.

Further described herein are methods for identifying a test compound that modulates conjugation of a ubiquitin-like polypeptide to a target protein. A method may comprise a)providing a mixture comprising the target protein and a fluorescently labeled ubiquitin-like polypeptide; and b) detecting the attachment of labeled ubiquitin-like polypeptide to the protein by fluorescence polarization in the presence and absence of the test compound, wherein if the fluorescence polarization of the labeled ubiquitin-like polypeptide in the presence of the compound is altered relative to the fluorescence polarization of the labeled ubiquitin-like polypeptide in the absence of the test compound, a test compound that modulates conjugation of the ubiquitin-like polypeptide to the target protein is identified. The ubiquitin-like polypeptide may be selected from the group consisting of: NEDD8, ISG15, SUMO1, SUMO2, SUMO3, APG12, and APGA8.

Also described herein are methods and reagents for evaluating the attachment of ubiquitin to a target protein, wherein an interaction between labeled ubiquitin and a second labeled protein (a recognition element) that binds the ubiquitin or binds the target protein, which may be ubiquitinated, results in an output signal when the fluorescently labeled ubiquitin comes into close proximity with the fluorescently labeled second protein. As an example, in a mixture comprising a target protein (such as an E3), a ubiquitin directly labeled with a fluorescent label (such as europium cryptate), and a fluorescently labeled antibody (such as an XL665-labeled antibody) that binds the E3, the attachment of the labeled ubiquitin to the E3 can be detected when the fluorescently-labeled ubiquitin comes into close proximity with the fluorescently labeled antibody, which will typically occur when both are bound to the E3. The interaction between the fluorescently labeled ubiquitin and fluorescently labeled antibody could be evaluated by, for example, time-resolved fluorescence resonance energy transfer (TR-FRET). Similarly, the interaction between a fluorescently labeled ubiquitin and a fluorescently labeled polyubiquitin-binding protein, such as S5a, TAB2, or TAB3, that is bound to the polyubiquitin conjugated to the target protein, such as a polyubiquitinated E3, could be evaluated by TR-FRET.

Also described herein are methods of evaluating the conjugation of a recognition element to a second polypeptide. A method may comprise: evaluating the rotational freedom of the recognition element in a mixture, wherein a decrease in rotational freedom of the recognition element relative to unconjugated recognition element indicates that the recognition element is conjugated to a second polypeptide. The recognition element may be labeled directly (e.g., with a fluorophore) or indirectly. Rotational freedom of the labeled recognition element may be detected by detecting fluorescence polarization. Evaluating the rotational freedom of recognition element in a mixture may comprise: a) providing a mixture comprising a ligase (a protein that mediates attachment of a ubiquitin or ubiquitin-like polypeptide to a substrate protein), a ubiquitin or ubiquitin-like polypeptide, and a recognition element; and b) detecting the rotational freedom of the recognition element. The mixture may further comprise a substrate of the ligase. The recognition element may be selected from the group consisting of: S5a, TAB2, and TAB3.

Further described herein are methods of detecting the attachment of ubiquitin or a ubiquitin-like polypeptide to a target protein. A method may comprise: detecting, in a mixture comprising the target protein, ubiquitin or a ubiquitin-like polypeptide, and a fluorescently labeled recognition element, the attachment of the labeled recognition element to the target protein by detecting fluorescence polarization. The mixture may further comprise a ligase polypeptide. The ubiquitin-like polypeptide may be directly labeled with a fluorophore. The target protein may be an E3, such as a POSH protein, a cbl-b protein or a PEM-3-like protein, or a portion thereof that is ubiquitinated. The attachment of more than one ubiquitin-like polypeptide to the target protein may be detected, and the target protein may itself be a ubiquitin-like polypeptide. Optionally, the target protein may be The recognition element may be selected from the group consisting of: S5a, TAB2, and TAB3.

In addition, described herein are methods for identifying a test compound that modulates conjugation of a ubiquitin polypeptide to a target protein. A method may comprise a) providing a mixture comprising the target protein, ubiquitin, and a fluorescently labeled recognition element; and b) detecting the attachment of labeled recognition element to the protein by fluorescence polarization in the presence and absence of the test compound, wherein if the fluorescence polarization of the labeled recognition element in the presence of the compound is altered relative to the fluorescence polarization of the labeled recognition element in the absence of the test compound, a test compound that modulates conjugation of the ubiquitin to the target protein is identified. The recognition element may be selected from the group consisting of: S5a, TAB2, and TAB3.

Also described herein are methods for detecting attachment of ubiquitin to a target protein. A method may comprise: detecting, in a mixture comprising the target protein, a fluorescently labeled ubiquitin, and a fluorescently labeled recognition element, the attachment of the labeled ubiquitin to the target protein by detecting an output signal when the fluorescently labeled ubiquitin comes into close proximity with the fluorescently labeled recognition element. In certain embodiments, the fluorescently labeled polypeptides exhibit FRET. The output signal may thus be a reduction in the intensity of the fluorescent signal from the fluorescent molecule that is a donor, reduction in the lifetime of its excited state, and/or re-emission of fluorescent light at the longer wavelengths (lower energies) characteristic of the fluorescent molecule that is the acceptor. The mixture may further comprise a ligase polypeptide. The ubiquitin may be directly labeled with a fluorophore. Optionally, the fluorophore may be a cryptate moiety, such as europium cryptate. The recognition element may be an antibody to the target polypeptide. In certain aspects, the antibody is directly labeled with a fluorescent label. Optionally, the fluorescent label is XL665. The recognition element may be selected from the group consisting of: S5a, TAB2, and TAB3. In certain embodiments, the recognition element is indirectly labeled with a fluorescent label. The target protein may be an E3, such as a POSH protein, a cbl-b protein or a PEM-3-like protein, or a portion thereof that is ubiquitinated. The attachment of more than one ubiquitin-like polypeptide to the target protein may be detected, and the target protein may itself be a ubiquitin-like polypeptide. In some embodiments, the target polypeptide is a polyubiquitinated E3.

Further embodiments will be understood from the description below.

BRIEF DESCRIPTION OF THE DRAWINGS

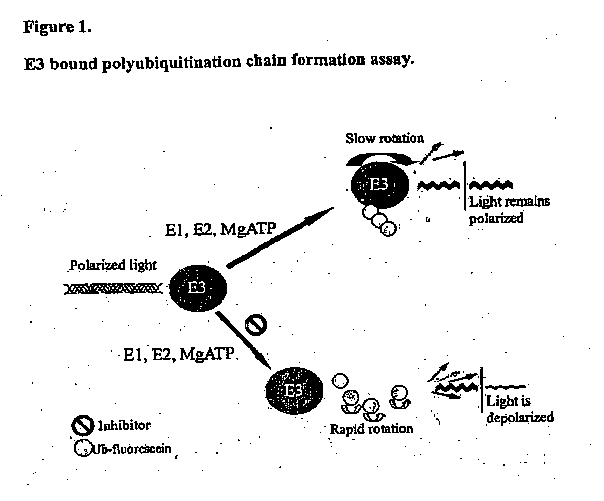

Figure 1 is a schematic depicting an assay for E3 bound polyubiquitination chain formation according to the present invention.

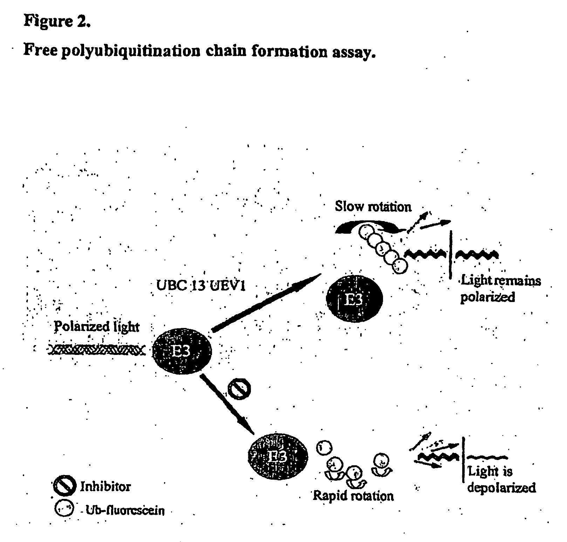

Figure 2 is a schematic depicting an assay for free polyubiquitination chain formation according to the present invention.

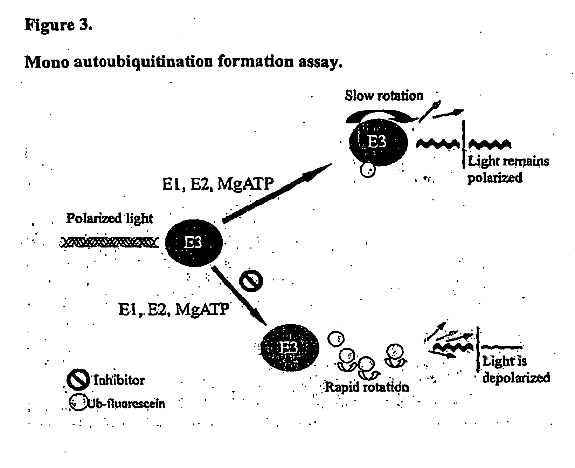

Figure 3 is a schematic depicting an assay for mono autoubiquitination formation according to the present invention.

Figure 4 is a schematic depicting an assay for polyubiquitination chain formation according to the present invention.

Figure 5 is a bar graph depicting a comparison between two types of ubiqutination assays. A) Background Control is determined as fluorescence obtained in parallel incubation without E3. B) Auto-ubiquitination is determined by TR-FRET. The conjugation of ubiquitin cryptate to hPOSH and the binding of anti-hPOSH antibody tagged XL665 bring the two fluorophores into close proximity, which allows the FRET reaction to occur. C) Ubiquitin polychain formation detection using S5a.

DETAILED DESCRIPTION OF THE INVENTION

In certain aspects, the invention relates to methods and compositions employing labeled ubiquitin ("Ub"), or ubiquitin-like ("Ubl"), polypeptides to evaluate the rotational freedom of the labeled Ub, or Ubl, polypeptide as characterised by the appended claims. In certain preferred embodiments, the Ub or Ubl, is labeled with a fluorophore, and the rotational freedom of the Ub or Ubl, is evaluated by fluorescence polarization. The Ub or Ubl polypeptide may be bound to the label (directly labeled) or may bind to a second molecule which is itself bound to the label (indirectly labeled).

Fluorescence polarization ("FP") or anisotropy is a highly sensitive method for detecting the rotational freedom of Ub or Ubl polypeptides according to the present invention. Briefly, when a fluorescent molecule is excited with a polarized light source, the molecule will emit fluorescent light in a fixed plane, e.g., the emitted light is also polarized, provided that the molecule is fixed in space. However, because the molecule is typically rotating and tumbling in space, the plane in which the fluoresced light is emitted varies with the rotation of the molecule (also termed the rotational diffusion of the molecule). However, if the molecule rotates or tumbles out of the plane of the exciting polarized light during the excited state, light is emitted in a different plane from that of the initial excitation. Restated, the emitted fluorescence is generally depolarized. The faster the molecule rotates in solution, the more depolarized it is. Conversely, the slower the molecule rotates in solution, the less depolarized, or the more polarized it is. The degree to which the fluorescence emission vector moves from, e.g., a vertical to a horizontal plane is directly related to the mobility of the fluorescently labeled molecule. That is, if the fluorescently labeled molecules are large, they move very little and the emitted light remains highly polarized with respect to the excitation plane. By contrast, if the fluorescently labeled molecules are small, they rotate or tumble faster, and the resulting emitted light is depolarized relative to the excitation plane (Lackowicz, Principles of Fluorescence Spectroscopy, Plenum Press, NY, 1983; Methods in Fluorescence Polarization, Panvera Corp, Madison Wis.).

The polarization value (P) for a given molecule is proportional to the molecule's "rotational correlation time," or the amount of time it takes the molecule to rotate through an angle of 57.3° (1 radian). The smaller the rotational correlation time, the faster the molecule rotates, and the less polarization will be observed. The larger the rotational correlation time, the slower the molecule rotates, and the more polarization will be observed. Rotational relaxation time is related to viscosity ($\eta$), absolute temperature (T), molar volume (V), and the gas constant (R). The rotational correlation time is generally calculated according to the following formula:

$$\text{Rotational Correlation Time} = (3\eta V)/(RT)$$

As can be seen from the above equation, if temperature and viscosity are maintained constant, then the rotational relaxation time, and therefore, the polarization value, is directly related to the molecular volume. Accordingly, the larger the molecule, the higher its fluorescent polarization value, and conversely, the smaller the molecule, the smaller its fluorescent polarization value.

Generally, the fluorescence polarization level is calculated using the following formula:

$$P = [I_{\|} - I_{\perp}]/[I_{\|} + I_{\perp}]$$

where $I_{\|}$ is the intensity of emission light parallel to the excitation plane, and $I_{\perp}$ is the intensity of emission light perpendicular to the excitation plane. P is a dimensionless number (expressed as "polarization units" or "millipolariztion units" (mP)).

Fluorescence anisotropy (r) is related to polarization (P) in the following way:

$$r = (I_{\|} - I_{\perp})/(I_{\|} + 2\,I_{\perp}) \text{ and } r = 2\,P/(3 - P).$$

Fluorescence polarization (P) and fluorescence anisotropy (r) are related and contain equivalent physical information with respect to monitoring macromolecular complex formation. Many instruments report on both polarization and anisotropy and either parameter can be used to evaluate Kd. Reference to a detection of "fluorescence polarization" herein is intended to mean detection of any physical parameter that permits the calculation of the polarization or anisotropy or other indicator of the degree of rotational freedom of the labeled Ub or Ubl polypeptide.

Fluorescence polarization/anisotropy is related to the speed at which a fluorescently labeled molecule rotates, which, in turn, is related to the size (molecular volume) of the fluorescent entity. In the performance of fluorescent binding assays, a fluorescently labeled molecule, e.g., ubiquitin, a ubiquitin-like polypeptide, a ligand, antigen, etc., having a relatively fast rotational correlation time, is used to bind to a larger molecule, e.g., an E3, a polyubiquitin chain, a receptor protein, antibody, etc., which has a slower rotational correlation time. The binding of the small labeled molecule to the larger molecule increases the rotational correlation time (decreases the amount of rotation) of the labeled species, namely the labeled complex over that of the free unbound labeled molecule. This has a corresponding effect on the level of polarization that is detectable. Specifically, the labeled complex presents much higher fluorescence polarization than the unbound, labeled molecule. Thus, when a fluorescently labeled Ub polypeptide, for example, binds to a target protein such as the E3, POSH, the molecular volume of the fluorescent entity increases, and, the fluorescence polarization value of the sample will be higher.

A particular reaction that is biologically or biochemically relevant, e.g., POSH autoubiquitination, may be carried out in the presence and absence of a compound that is to be screened, and the effect of the compound is determined. Specifically, if the reaction is slowed or blocked by the presence of the test compound, then the compound is identified as an inhibitor of the reaction. Conversely where the reaction proceeds more rapidly or to a greater extent in the presence of the test compound, then the compound is identified as an enhancer of the reaction. These screening assays are then performed for a large number of different compounds, either serially or in parallel, in order to expedite the discovery of potential effectors of the reaction of interest.

In performing screening assays according to certain embodiments of the subject application, e.g., for potential inhibitors or enhancers of ubiquitination, the fluorescence polarization of the reaction mixture is compared in the presence and absence of different compounds, to determine whether these different compounds have any effect on the binding function of interest. For example, in the presence of inhibitors of E3 activity, the fluorescence polarization will decrease, as more free, labeled ubiquitin is present in the assay. Conversely, enhancers of E3 activity will result in an increase in the fluorescence polarization, as more complexed and less free, labeled ubiquitin are present in the assay.

In another embodiment, for example, attachment of fluorescently labeled Ub to a target protein, such as POSH, is allowed to proceed in the absence and presence of test compounds (e.g., in control and test combinations, respectively), and fluorescence polarization measurements are used to quantify the level of ubiquitination in test and control combinations. Compounds that inhibit POSH autoubiquitination can thus be identified as those that cause a depolarization of the test combination relative to the control combination.

Fluorescence polarization measurements have long been a valuable biophysical research tool for investigating processes such as membrane lipid mobility, myosin reorientation and protein-protein interactions at the molecular level. Immunoassays that have been developed and used extensively for clinical diagnostics represent the largest group of bioanalytical applications. The more recent advent of microplate readers equipped with polarizing optics has led to the adoption of fluorescence polarization as a readout mode for high-throughput screening. Some typical bioanalytical applications of fluorescence polarization-based assays include ligand binding to neurokinin 1, (Tota MR, et al, 1994), ligand binding to melanocortin G-protein-coupled receptors, (Banks P J, et al 2000), ligand binding to B2 bradykinin receptor, a G-protein-coupled receptor, (Banks P J, et al, 2002), ligand binding to estrogen receptors, (Parker GJ , et al, 2000), ligand binding to tyrosine kinase Src homology domains, (Lynch BA , et al, 1999), substrate binding to protein farnesyltransferase, (Rozema DB, et al, 1999), lactam antibiotic binding to penicillin-binding proteins, (Zhao G , et al, 1999), protein kinase activity, (Coffin J, et al, 2000); detection of specific PCR products, (Kido C, et al, 2000), ligation and cleavage of RNA by ribozymes, (Singh KK, et al, 2000), and protein-protein and protein-nucleic acid interactions, (Rusinova E, et al, 2002).

Techniques to use FP for the study of protein-protein interaction are reviewed in e.g. Nasir et al. (1999) Combinatorial Chemistry & High Throughput Screening, 2: 177-190, and Lundblad et al. (1996) Mol. Endocrinol. 10: 607-612. Furthermore, WO 00/50896 teaches methods and compositions to assess enzyme-mediated interaction of binding partners, i.e. addition or subtraction of a chemical moiety to or from one of the binding partners by a protein modifying enzyme, and hints that e.g. ubiquitin modification by E3 ligase may be monitored. US 2002/0194606 discloses that fluorescence polarization may be employed to quantitatively measure the effect of a modification enzyme, such as an enzyme transferring short peptides.

FP is used to determine if a fluorescently labeled small molecule binds to a much larger binding molecule, such as an antibody, a nucleic acid sequence, an enzyme, a receptor or a binding protein with certain specificity (see, e.g., PCT publication WO 98/18956; PCT publication WO 98/18956; PCT publication WO 98/05962; U.S. Pat. No. 6,326,142; U.S. Pat. No. 6,100,039; and U.S. Pat. No. 6,202,397). FP has also been used to determine if a fluorescently labeled compound is degraded or digested or if a fluorescently labeled molecule is incorporated into a larger molecule [e.g., see U.S. Pat. No. 5,786,139; T. M. Hsu et al., Biotechniques, 31, pp. 560-68 (2001); P. Y. Kwok, Hum. Mutat., 19, pp.315-23 (2002)]

One advantage of FP is that it can be utilized in homogeneous assays, such as high throughput screening assays. FP is also amenable to performing assays in real-time, directly in solution and without the need for an immobilized phase. Polarization values can be measured repeatedly and after the addition of reagents since measuring the samples is rapid and does not destroy the sample.

Fluorescence Polarization can be used to provide a direct, nearly instantaneous measure of a tracer's bound/free ratio. FP experiments are generally done in solution without solid supports, allowing true equilibrium analysis down to the low picomolar range. FP measurements do not usually adulterate samples, so they can be treated and reanalyzed in order to ascertain the effect on binding by changes such as pH, temperature, and salt concentration. Additionally, FP experiments are taken in "real-time" and experiments are not limited to equilibrium binding studies.

Fluorescence polarization may be designed to offer numerous advantages over more conventional methods to study the binding of proteins to nucleic acids (particularly in that no hazardous radioactive waste is generated) and as noted above, has a lower limit of detection in the sub-nanomolar range. FP is furthermore homogeneous, allows real-time measurements (kinetic assays), is insensitive to variations in concentrations and is an optimal solution for homogeneous assay formats (no separation by washing). (Handbook of Fluorescent Probes and Research Reagent-Molecular Probes, Haugland RP, 2003; High through put screening, methods and protocols , Janzen PW Humana press).

As noted above, the assay methods of the present application typically utilize a Ub or Ubl polypeptide that is directly labeled, meaning that the Ub or Ubl is attached to or includes a fluorescent labeling group. Also described herein are assay methods that utilize a recognition element, such as a GST-tagged S5a polypeptide, that is indirectly labeled (e.g., by binding to a labeled anti-GST antibody or other labeled binding protein). The fluorescent label on the first reagent may be selected from any of a variety of different fluorescent labeling compounds as described herein. Fluorescent labeling materials are commercially available from, e.g., Molecular Probes (Eugene, Oreg.). Typically, fluorescein or rhodamine derivatives are particularly well suited to the assay methods employing fluorescence polarization described herein. These fluorescent labels are coupled to the first reagent, e.g., covalently through well known coupling chemistries. For a discussion of labeling groups and chemistries, see, e.g., Published International Patent Application No. WO 98/00231.

A relatively small fluorescent compound, e.g., a labeled ubiquitin, generally emits relatively depolarized fluorescence when it is excited by polarized excitation light This is generally due to the faster rotational diffusion or "spin" of these smaller molecules. Larger molecules on the other hand have slower spin and thus are more likely to emit relatively polarized fluorescence when excited by a polarized excitation light source. Typically, the detected fluorescence polarization, or P value, provides a measure of the ratio of bound label to free label, although assay results may also be determined as a difference between pre-reaction fluorescence polarization and post-reaction fluorescence polarization, with the difference being an indication of the reaction's rate and/or completeness. The level of fluorescence polarization of the product then provides an indication of the amount of the fluorescent label that is bound to the larger molecule, e.g., as the ratio of bound to free label. Typically, fluorescence polarization data are generally reported as the ratio of the difference of parallel and perpendicular fluorescence emissions to the sum of these fluorescent emissions. Thus, the smaller the difference between these fluorescence emissions, e.g., the more depolarized the emissions, the smaller the polarization value. Conversely, more polarized emissions yield larger numbers. As alluded to above, in comparing assay results, the polarization value (P) for the reaction mix is determined. The fraction of bound fluorescence, e.g., associated with the polyion, is determined as:

$$F_b = (P - P_f)/(P_b - P_f)$$

where $P_b$ is the P value of the bound species, and $P_f$ is the P value of the free species. Thus, the polarization value can be used as an absolute quantitative measurement of the ratio of product to substrate, where one has determined or is already aware of the P value for completely bound label and completely free label. Alternatively, as noted above, one can measure the pre-reaction and post reaction fluorescence polarization, using the difference between the two as an indication of the amount of product produced.

The P value serves as an indicator of the reaction of interest, e.g., by indicating the amount of product produced. Once an assay reaction is quantifiable, one can use that assay in a number of different applications, including for example diagnostics, but particularly for screening of potential inhibitors or enhancers of the reaction of interest. This is typically useful in screening compound libraries against pharmacologically relevant targets that utilize one or more of the reactions described herein, e.g., attachment of ubiquitin or Ubl polypeptides to a protein of interest, such as POSH.

Although generally described in terms of detection of fluorescent polarization, it will be readily appreciated that a variety of detection schemes may be employed that detect the rate of rotation of a molecule or the translation or lateral diffusion of a molecule that relates to the size of the molecule. Examples of methods of detecting a molecule's rotation include, e.g., nuclear magnetic resonance spectroscopy, electron spin resonance spectroscopy, and triplet state absorbance anisotropy. Examples of methods of detecting the translation rate of molecules include, e.g., fluorescent correlation spectroscopy, fluorescence recovery after photobleaching, and magnetic resonance spin exchange spectroscopies. The assay methods of the present application may utilize a Ub or Ubl polypeptide that includes a fluorescent labeling group or any other labeling group suitable for detecting the rotational freedom of the Ub or Ubl polypeptide.

Specific applications to which the assays of the present application are put include the ability to test the effects of potential pharmaceutical candidate compounds on the various activities described above. For example, in pharmaceutical discovery processes, large libraries of chemical compounds are generally screened against pharmacologically relevant targets. These targets may include receptors, enzymes, transporters, and the like. A variety of screening assays and systems have been described. See, e.g., Published International Patent Application No. WO 98/00231.

An overall assay system for use in practicing the present invention includes a reaction receptacle. A detector or detection system is disposed adjacent to the receptacle and within sensory communication of the receptacle. The phrase "within sensory communication" generally refers to the detector that is positioned relative to the receptacle so as to be able to receive a particular signal from that receptacle. In the case of optical detectors, e.g., fluorescence or fluorescence polarization detectors, sensory communication typically means that the detector is disposed sufficiently proximal to the receptacle that optical, e.g., fluorescent signals are transmitted to the detector for adequate detection of those signals. Typically this employs a lens, optical train or other detection element, e.g., a CCD, that is focused upon a relevant portion of the receptacle to efficiently gather and record these optical signals.

A detector is typically connected to an appropriate data storage and/or analysis unit, e.g., a computer or other processor, which is generally capable of storing, analyzing and displaying the obtained data from the receptacle in a user comprehendible fashion, e.g., a display. In certain embodiments, e.g., those employing microfluidic receptacles, the computer is optionally connected to an appropriate controller unit, which controls the movement of fluid materials within the channels of the microfluidic device receptacle, and/or controls the relative position of the receptacle and detector, e.g., via an x-y-z translation stage. The receptacle also typically includes a detection zone as well as a detector disposed in sensory communication with the detection zone. The detector used in accordance with the present invention typically is configured to detect a level of fluorescence polarization of reagents in the detection zone.

As used herein, the receptacle may take on a variety of forms. For example, the receptacle may be a simple reaction vessel, well, tube, cuvette, or the like. Alternatively, the receptacle may comprise a capillary or channel either alone or in the context of an integrated fluidic system that includes one or more fluidic channels, chambers or the like.

In embodiments relating to detection of fluorescence polarization, the methods and systems of the present invention typically rely upon a change in the level of fluorescence polarization of the reaction mixture as a result of the reaction of interest. As such, an appropriate detection system is typically utilized to differentiate polarized from depolarized emitted fluorescence. Generally speaking, such a detection system typically separately detects fluorescent emissions that are emitted in the same plane of the polarized excitation light, and fluorescent emissions emitted in a plane other than the plane of the excitation light.

One example of a detection system is a fluorescence polarization detector that includes a light source, which generates light at an appropriate excitation wavelength for the fluorescent compounds that are present in the assay system. Typically, coherent light sources, such as lasers, laser diodes, and the like are preferred because of the highly polarized nature of the light produced thereby. The excitation light is directed through an optional polarizing filter, which passes only light in one plane, e.g., polarized light. The polarized excitation light is then directed through an optical train, e.g., dichroic mirror and microscope objective (and optionally, a reference beam splitter), which focuses the polarized light onto the sample receptacle in which the sample to be assayed is disposed.

Fluorescence emitted from the sample is then collected, e.g., through the objective, and directed back through the dichroic mirror, which passes the emitted fluorescence and reflects the reflected excitation light, thereby separating the two. The emitted fluorescence is then directed through a beam splitter where one portion of the fluorescence is directed through a filter that filters out fluorescence that is in the plane that is parallel to the plane of the excitation light and directs the perpendicular fluorescence onto a first light detector. The other portion of the fluorescence is passed through a filter that filters out the fluorescence that is perpendicular to the plane of the excitation light, directing the parallel fluorescence onto a second light detector. In alternative aspects, a beam splitter is substituted with a polarizing beam splitter, e.g., a Glan prizm, obviating the need for the filters described above. These detectors are then typically coupled to an appropriate recorder or processor where the light signal is recorded and or processed as set out in greater detail below. Photomultiplier tubes (PMTs), are generally preferred as light detectors for the quantification of the light levels, but other light detectors are optionally used, such as photodiodes, or the like.

The detector is typically coupled to a computer or other processor, which receives the data from the light detectors, and includes appropriate programming to compare the values from each detector to determine the amount of polarization from the sample. In particular, the computer typically includes software programming which receives as input the fluorescent intensities from each of the different detectors, e.g., for parallel and perpendicular fluorescence. The fluorescence intensity is then compared for each of the detectors to yield a fluorescence polarization value. One example of such a comparison is given by the equation:

$$P = [I_\| - I^\perp]/[ I_\| + I^\perp] C$$

as shown above, except including a correction factor (C), which corrects for polarization bias of the detecting instrument. The computer determines the fluorescence polarization value for the reaction of interest. From that polarization value and based upon the polarization values for free and bound fluorescence, the computer calculates the ratio of bound to free fluorescence. Alternatively, the polarization values pre and post reaction are compared and a polarization difference (ΔP) is determined. The calculated polarization differences may then be used as absolute values, e.g., to identify potential effectors of a particular reaction, or they may be compared to polarization differences obtained in the presence of known inhibitors or enhancers of the reaction of interest, in order to quantify the level of inhibition or enhancement of the reaction of interest by a particular compound.

Examples of instruments that may be used to determine fluorescence polarization in the methods of the present application include the Beacon® 2000 Fluorescence Polarization System (Invitrogen), the Ultra Evolution (Tecan), and Analyst AD (Molecular Devices).

Also described herein is that the formation of ubiquitin complexes may be measured by an interactive technique, such as FRET, wherein a ubiquitin is labeled with a first label and the desired complex partner (e.g., POSH polypeptide or target polypeptide) is labeled with a second label, wherein the first and second label interact when they come into close proximity to produce an altered signal. In FRET, the first and second labels are fluorophores. The formation of polyubiquitin complexes may be performed by mixing two or more pools of differentially labeled ubiquitin that interact upon formation of a polyubiquitin (see, e.g. US Patent Publication 20020042083). High-throughput may be achieved by performing an interactive assay, such as FRET, in solution as well. In addition, if a polypeptide in the mixture, such as the POSH polypeptide or target polypeptide, is readily purifiable (e.g., with a specific antibody or via a tag such as biotin, FLAG, polyhistidine, etc.), the reaction may be performed in solution and the tagged polypeptide rapidly isolated, along with any polypeptides, such as ubiquitin, that are associated with the tagged polypeptide. Proteins may also be resolved by SDS-PAGE for detection.

Fluorescence Resonance Energy Transfer (FRET)-based assays may be used to determine complex formation. Fluorescent molecules having the proper emission and excitation spectra that are brought into close proximity with one another can exhibit FRET. The fluorescent molecules are chosen such that the emission spectrum of one of the molecules (the donor molecule) overlaps with the excitation spectrum of the other molecule (the acceptor molecule). The donor molecule is excited by light of appropriate intensity within the donor's excitation spectrum. The donor then emits the absorbed energy as fluorescent light. The fluorescent energy it produces is quenched by the acceptor molecule. FRET can be manifested as a reduction in the intensity of the fluorescent signal from the donor, reduction in the lifetime of its excited state, and/or re-emission of fluorescent light at the longer wavelengths (lower energies) characteristic of the acceptor. When the fluorescent proteins physically separate, FRET effects are diminished or eliminated. (U.S. Patent No. 5,981,200).

For example, a cyan fluorescent protein is excited by light at roughly 425 - 450 nm wavelength and emits light in the range of 450 - 500 nm. Yellow fluorescent protein is excited by light at roughly 500 - 525 nm and emits light at 525 - 500 nm. If these two proteins are placed in solution, the cyan and yellow fluorescence may be separately visualized. However, if these two proteins are forced into close proximity with each other, the fluorescent properties will be altered by FRET. The bluish light emitted by CFP will be absorbed by YFP and re-emitted as yellow light. This means that when the proteins are stimulated with light at wavelength 450 nm, the cyan emitted light is greatly reduced and the yellow light, which is not normally stimulated at this wavelength, is greatly increased. FRET is typically monitored by measuring the spectrum of emitted light in response to stimulation with light in the excitation range of the donor and calculating a ratio between the donor-emitted light and the acceptor-emitted light. When the donor/acceptor emission ratio is high, FRET is not occurring and the two fluorescent proteins are not in close proximity. When the donor: acceptor emission ratio is low, FRET is occurring and the two fluorescent proteins are in close proximity. In this manner, the interaction between a first and second polypeptide may be measured.

The occurrence of FRET also causes the fluorescence lifetime of the donor fluorescent moiety to decrease. This change in fluorescence lifetime can be measured using a technique termed fluorescence lifetime imaging technology (FLIM) (Verveer et al. (2000) Science 290: 1567-1570; Squire et al. (1999) J. Microsc. 193: 36; Verveer et al. (2000) Biophys. J. 78: 2127). Global analysis techniques for analyzing FLIM data have been developed. These algorithms use the understanding that the donor fluorescent moiety exists in only a limited number of states each with a distinct fluorescence lifetime. Quantitative maps of each state can be generated on a pixel-by-pixel basis.

Further described herein is the use of time-resolved fluorescence resonance energy transfer (TR-FRET). TR-FRET is based on the proximity of a donor label (such as europium chelate) and an acceptor label (such as allophycocyanin), which have been brought together by a specific binding reaction. The excited energy of the Eu-chelate is transferred by a nonradiative resonance energy transfer mechanism to an acceptor within a short distance. Fluorescent lanthanide chelates with long excited state lifetimes are typically used to avoid interference caused by short-lived emission from acceptor molecules excited directly rather than by energy transfer.

Other donor and acceptor species suitable for use in TR-FRET include the use of a long-lifetime terbium chelate as the donor species and fluorescein as the acceptor species. When terbium and fluorescein labeled molecules are brought into proximity, energy transfer takes place causing an increase in acceptor fluorescence and a decrease in donor fluorescence. These fluorescent signals can be read in a time-resolved manner.

Also descibed herein is that the fluorescent label donor is europium cryptate, and the fluorescent label acceptor is a modified allophycocyanin, a crosslinked 105 kDa phycobiliprotein, known as XL665.

FRET-based assays may be used in cell-based assays and in cell-free assays. FRET-based assays are amenable to high-throughput screening methods including Fluorescence Activated Cell Sorting and fluorescent scanning of microtiter arrays.

Further described herein are methods and systems relating to ubiquitin and ubiquitin-like protein modification systems. A ubiquitinated protein substrate is a protein complex comprising ubiquitin covalently attached to the protein substrate. Therefore, methods and systems are described herein that utilize a ubiquitination machinery and identify specific protein substrates that are ubiquitinated by the machinery, and methods and systems are based on the ubiquitination machinery- or E3-mediated protein-protein interaction between ubiquitin (or another protein modifier) and its protein substrate. Likewise, methods described herein may be used to analyze the interaction between a known E3 and a known substrate, or to identify an E3 for a protein of interest. It is noted that the E3-mediated protein-protein interaction described herein may be distinct from a simple tripartite or ternary protein complex in that E3, acting as an enzyme, catalyzes the protein-protein interaction which leads to a ubiquitinated (or similarly modified) protein substrate. Certain methods and systems described herein are further suitable to conduct high throughput screening, for example, to identify protein substrates subject to ubiquitination or other protein modification.

Naturally occurring ubiquitin, or "Ub," as used herein refers to an abundant 76 amino acid residue polypeptide that is found in most, if not all, eukaryotic cells. The Ub polypeptide is characterized by a carboxy-terminal glycine residue that is activated by ATP to a high-energy thiol-ester intermediate in a reaction catalyzed by a Ub-activating enzyme (E1). The activated Ub is transferred to a substrate polypeptide via an isopeptide bond between the activated carboxy-terminus of Ub and the epsilon-amino group of a lysine residue(s) in the protein substrate. This transfer requires the action of Ub conjugating enzymes such as E2 and, in some instances, auxiliary substrate recognition or Ub ligase (E3) activities. The Ub-modified substrate is thereby altered in biological function, and, in some instances, becomes a substrate for components of the Ub-dependent proteolytic machinery which includes both Ub isopeptidase enzymes as well as proteolytic proteins which are subunits of the proteasome. As used herein, the term "ubiquitin" or Ub includes within its scope all known as well as unidentified eukaryotic Ub homologs of vertebrate or invertebrate origin. Examples of Ub polypeptides as referred to herein include the human Ub polypeptide that is encoded by the human Ub encoding nucleic acid sequence (GenBank Accession Numbers: U49869, X04803) as well as all equivalents. Another example of a Ub polypeptide as referred to herein is murine Ub which is encoded by the murine Ub nucleic acid coding sequence (GenBank Accession Number: X51730).

The term "ubiquitin-like" or "Ubl" protein modifiers as used herein refer to the group of small proteins that are subject to conjugation machinery similar to that for ubiquitination. Examples of Ubl protein modifiers include NEDD8, ISG15; SUMO 1 , SUMO2, SUMO3, APG12, APG8, as fisted in Wong et al., *supra,* as well as other Ubl protein modifiers yet to be identified. An example of a Ubl polypeptide as referred to herein is murine SUMO1 (also termed GMP1, Picl, SMTP3, Smt3C, sentrin) which is encoded by the murine encoding nucleic acid sequence (GenBank Accession Number: NM_009460).

The term antibody as used herein is intended to include fragments thereof which are also specifically reactive with one of the subject target polypeptides, which is polyubiquitinated or may be polyubiquinated. Antibodies can be fragmented using conventional techniques and the fragments screened for utility in the same manner as described above for whole antibodies. For example, $F(ab)_2$ fragments can be generated by treating antibody with pepsin. The resulting $F(ab)_2$ fragment can be treated to reduce disulfide bridges to produce Fab fragments. The term antibody is further intended to include bispecific, single-chain, and chimeric and humanized molecules having affinity for a target polypeptide conferred by at least one CDR region of the antibody. In preferred embodiments, the antibody further comprises a label attached thereto and able to be detected (e.g., the label can be a radioisotope, fluorescent compound, enzyme or enzyme co-factor).

The term "Ub or Ubl conjugation machinery" or "ubiquitination machinery" as used herein refers to a group of proteins which function in the ATP-dependent activation and transfer of Ub or Ubl to substrate proteins. The term thus encompasses: E1 enzymes, which transform the carboxy-terminal glycine of Ub or Ubl into a high energy thiol intermediate by an ATP-dependent reaction; E2 enzymes (the LTBC genes), which transform the El-S-Ub/Ubl activated conjugate into an E2S-Ub/Ubl intermediate which acts as a Ub or Ubl donor to a substrate, another Ub moiety (in a poly-ubiquitination reaction), or an E3; and the E3 enzymes which facilitate the transfer of an activated Ub or Ubl molecule from an E2 to a substrate molecule or to another Ub or Ubl moiety as part of a polyubiquitin chain. The term "Ub or Ubl conjugation machinery" or ubiquitination machinery as used herein, is further meant to include all known members of these groups as well as those members which have yet to be discovered or characterized but which are sufficiently related by homology to known Ub or Ubl conjugation enzymes so as to allow an individual skilled in the art to readily identify it as a member of this group. The term as used herein is meant to include novel Ub activating enzymes (E2s) which have yet to be discovered as well as those which function in the activation and conjugation of Ubl or Ub-related polypeptides to their substrates and to poly-Ubl or poly-Ub-related protein chains.

Essentially any E3 may be used in methods and systems disclosed herein. For example, Wong et al. discloses four subclasses of E3s: RING, PHD, HECT, and U-box. The RING subclass comprises 439 isoforms (e.g., alternative splicing variants), the PHD subclass 137 isoforms, the HECT subclass 43 isoforms, and the U-box subclass 13 isoforms. Yet other new E3 proteins or isoforms may be discovered. As used herein, the term "E3" or "E3 protein" is intended to encompass any portion of an E3 that is sufficient to mediate ubiquitination of a substrate protein. An E3 may also comprise more than one polypeptide or fragments of polypeptides.

An example of an E3 for use in the methods and systems of the invention is POSH (Plenty Of SH3 domains) nucleic acid sequences and proteins encoded thereby. POSH comprises a RING domain and undergoes a self-mediated ubiquitination. POSH proteins play a role in viral maturation, protein trafficking and other significant biological processes. For example, POSH may act in the assembly or trafficking of complexes that mediate viral release. Many features of POSH, and particularly human POSH, are described in PCT patent publications WO03/095971A2 (application no. WO2002US0036366) and WO03/078601A2 (application no. WO2003US0008194).

"Homology" or "identity" or "similarity" refers to sequence similarity between two peptides or between two nucleic acid molecules. Homology can be determined by comparing a position in each sequence which may be aligned for purposes of comparison. Preferably, such comparisons will be made using the well-known BLAST algorithm. When a position in the compared sequence is occupied by the same base or amino acid, then the molecules are identical at that position. A degree of homology or similarity or identity between nucleic acid sequences is a function of the number of identical or matching nucleotides at positions shared by the nucleic acid sequences. A degree of identity of amino acid sequences is a function of the number of identical amino acids at positions shared by the amino acid sequences. A degree of homology or similarity of amino acid sequences is a function of the number of amino acids, i.e. structurally related, at positions shared by the amino acid sequences. An "unrelated" or "nonhomologous" sequence shares less than 40 % identity, though preferably less than 25 % identity, with one of the E3 sequences of the present invention.

It will be generally appreciated that, under certain, circumstances, it may be advantageous to provide homologs of an E3 or Ub or Ubl polypeptide or recognition element of the invention. For example, such homologs may be useful when, e.g., the E3 or Ub or Ubl also comprises an undesirable biological activity to a host cell of the invention. Thus, an E3 or Ub or Ubl derived from the nonnaturally occurring homologs may be used to practice the present invention with fewer side effects relative to an E3 or Ub or Ubl derived from the naturally occurring polypeptides. Accordingly, the terms "E3," "Ub," "Ubl," and "recognition element" are intended to encompass such homologs thereof.

Homologs of each of the subject subunit polypeptides can be generated by mutagenesis, such as by discrete point mutation(s), or by truncation. WO0022110 describes various methods to create polypeptide homologs.

The terms "protein," "polypeptide" and "peptide" are used interchangeably herein when referring to a gene product. The terms refer to polymers of amino acid of any length. The polymer may be linear or branched, it may comprise modified amino acids, and it may be interrupted by non-amino acids. It also may be modified naturally or by intervention, for example, disulfide bond formation, glycosylation, myristoylation, acetylation, alkylation, phosphorylation or dephosphorylation. Also included within the definition are polypeptides containing one or more analogs of an amino acid (including, for example, unnatural amino acids) as well as other modifications known in the art.

As used herein, the term "nucleic acid" refers to polynucleotides such as DNA, and, where appropriate, ribonucleic acid (RNA). The term should also be understood to include analogs of either RNA or DNA made from nucleotide analogs, and, as applicable to the embodiment being described, single (sense or antisense) and double-stranded polynucleotides.

For convenience, certain terms employed in the specification, examples, and appended claims are collected here. Unless defined otherwise, all technical and scientific terms used herein have the same meaning as commonly understood by one of ordinary skill in the art to which this invention belongs.

The articles "a" and "an" are used herein to refer to one or to more than one (i.e., to at least one) of the grammatical object of the article, unless context clearly indicates otherwise. By way of example, "an element" means one element or more than one element.

The term "including" is used herein to mean, and is used interchangeably with, the phrase "including but not limited to".

The term "or" is used herein to mean, and is used interchangeably with, the term "and/or", unless context clearly indicates otherwise.

In certain embodiments, an assay comprises forming a mixture comprising a POSH polypeptide, an E2 polypeptide and a source of labeled ubiquitin. Optionally the mixture comprises an E1 polypeptide and optionally the mixture comprises a target polypeptide. Additional components of the mixture may be selected to provide conditions consistent with the ubiquitination of the POSH polypeptide. One or more of a variety of parameters may be detected, such as POSH-ubiquitin conjugates, E2-ubiquitin thioesters, free ubiquitin and target polypeptide-ubiquitin complexes. The term "detect" is used herein to include a determination of the presence or absence of the subject of detection (e.g., POSH-ubiquitin, E2-ubiquitin, etc.), a quantitative measure of the amount of the subject of detection, or a mathematical calculation of the presence, absence or amount of the subject of detection, based on the detection of other parameters. The term "detect" includes the situation wherein the subject of detection is determined to be absent or below the level of sensitivity. Detection may comprise detection of a label (e.g., fluorescent label, radioisotope label, and other described below), resolution and identification by size (e.g., SDS-PAGE, mass spectroscopy), purification and detection, and other methods that, in view of this specification, will be available to one of skill in the art. For instance, radioisotope labeling may be measured by scintillation counting, or by densitometry after exposure to a photographic emulsion, or by using a device such as a Phosphorimager. Likewise, densitometry may be used to measure bound ubiquitin following a reaction with an enzyme label substrate that produces an opaque product when an enzyme label is used. In some embodiments, an assay comprises detecting the POSH-ubiquitin conjugate.

In certain embodiments, an assay comprises forming a mixture comprising a POSH polypeptide, a target polypeptide and a source of labeled ubiquitin. Optionally the mixture comprises an E1 and/or E2 polypeptide and optionally the mixture comprises an E2-ubiquitin thioester. Additional components of the mixture may be selected to provide conditions consistent with the ubiquitination of the target polypeptide. One or more of a variety of parameters may be detected, such as POSH-ubiquitin conjugates and target polypeptide-ubiquitin conjugates. In some embodiments, an assay comprises detecting the target polypeptide-ubiquitin conjugate. In another embodiment, an assay comprises detecting the POSH-ubiquitin conjugate.

An assay described above may be used in a screening assay to identify agents that modulate a ubiquitin-related activity of a POSH polypeptide or other E3 ubiquitin ligase. A screening assay will generally involve adding a test agent to one of the above assays, or any other assay designed to assess a ubiquitin-related activity of a POSH polypeptide. The parameter(s) detected in a screening assay may be compared to a suitable reference. A suitable reference may be an assay run previously, in parallel or later that omits the test agent. A suitable reference may also be an average of previous measurements in the absence of the test agent In general the components of a screening assay mixture may be added in any order consistent with the overall activity to be assessed, but certain variations may be preferred. For example, in certain embodiments, it may be desirable to pre-incubate the test agent and the E3 (e.g., the POSH polypeptide), followed by removing the test agent and addition of other components to complete the assay. In this manner, the effects of the agent solely on the POSH polypeptide may be assessed. In certain embodiments, a screening assay for an antiviral agent employs a target polypeptide comprising an L domain, and preferably an HIV L domain.

In certain embodiments, an assay is performed in a high-throughput format. For example, one of the components of a mixture may be affixed to a solid substrate and one or more of the other components is labeled. For example, the POSH polypeptide may be affixed to a surface, such as a 96-well plate, and the ubiquitin is in solution and labeled. An E2 and E1 are also in solution, and the POSH-ubiquitin conjugate formation may be measured by washing the solid surface to remove uncomplexed labeled ubiquitin and detecting the ubiquitin that remains bound. Other variations may be used. For example, the amount of ubiquitin in solution may be detected.

In certain embodiments, the ubiquitin is labeled, either directly or indirectly. This typically allows for easy and rapid detection and measurement of ligated ubiquitin, making the assay useful for high-throughput screening applications. As described above, certain embodiments may employ one or more tagged or labeled proteins. A "tag" is meant to include moieties that facilitate rapid isolation of the tagged polypeptide. A tag may be used to facilitate attachment of a polypeptide to a surface. A "label" is meant to include moieties that facilitate rapid detection of the labeled polypeptide. Certain moieties may be used both as a label and a tag (e.g., epitope tags that are readily purified and detected with a well-characterized antibody). Biotinylation of polypeptides is well known, for example, a large number of biotinylation agents are known, including amine-reactive and thiol-reactive agents, for the biotinylation of proteins, nucleic acids, carbohydrates, carboxylic acids; see chapter 4, Molecular Probes Catalog, Haugland, 6th Ed. 1996. A biotinylated substrate can be attached to a biotinylated component via avidin or streptavidin. Similarly, a large number of haptenylation reagents are also known.

An "E1" is a ubiquitin activating enzyme. In a preferred embodiment, E1 is capable of transferring ubiquitin to an E2. In a preferred embodiment, E1 forms a high energy thiolester bond with ubiquitin, thereby "activating" the ubiquitin. An "E2" is a ubiquitin carrier enzyme (also known as a ubiquitin conjugating enzyme). In a preferred embodiment, ubiquitin is transferred from E1 to E2. In a preferred embodiment, the transfer results in a thiolester bond formed between E2 and ubiquitin. In a preferred embodiment, E2 is capable of transferring ubiquitin to a POSH polypeptide.

In an alternative embodiment, a POSH polypeptide, E2 or target polypeptide is bound to a bead, optionally with the assistance of a tag. Following ligation, the beads may be separated from the unbound ubiquitin and the bound ubiquitin measured. In a preferred embodiment, POSH polypeptide is bound to beads and the composition used includes labeled ubiquitin. In this embodiment, the beads with bound ubiquitin may be separated using a fluorescence-activated cell sorting (FACS) machine. Methods for such use are described in U.S. patent application Ser. No. 09/047,119. The amount of bound ubiquitin can then be measured.

In a screening assay, the effect of a test agent may be assessed by, for example, assessing the effect of the test agent on kinetics, steady-state and/or endpoint of the reaction.

The components of the various assay mixtures provided herein may be combined in varying amounts. In a preferred embodiment, ubiquitin (or E2 complexed ubiquitin) is combined at a final concentration of from 5 to 200 ng per 100 microliter reaction solution. Optionally E1 is used at a final concentration of from 1 to 50 ng per 100 microliter reaction solution. Optionally E2 is combined at a final concentration of 10 to 100 ng per 100 microliter reaction solution, more preferably 10-50 ng per 100 microliter reaction solution. In a preferred embodiment, POSH polypeptide is combined at a final concentration of from 1 ng to 500 ng per 100 microliter reaction solution.

Generally, an assay mixture is prepared so as to favor ubiquitin ligase activity and/or ubiquitination activity. Generally, this will be physiological conditions, such as 50 - 200 mM salt (e.g., NaCl, KCl), pH of between 5 and 9, and preferably between 6 and 8. Such conditions may be optimized through trial and error. Incubations may be performed at any temperature which facilitates optimal activity, typically between 4 and 40°C. Incubation periods are selected for optimum activity, but may also be optimized to facilitate rapid high through put screening. Typically between 0.5 and 1.5 hours will be sufficient. A variety of other reagents may be included in the compositions. These include reagents like salts, solvents, buffers, neutral proteins, e.g. albumin, detergents, etc. which may be used to facilitate optimal ubiquitination enzyme activity and/or reduce non-specific or background interactions. Also reagents that otherwise improve the efficiency of the assay, such as protease inhibitors, nuclease inhibitors, anti-microbial agents, etc., may be used. The compositions will also preferably include adenosine tri-phosphate (ATP). The mixture of components may be added in any order that promotes ubiquitin ligase activity or optimizes identification of candidate modulator effects. In a preferred embodiment, ubiquitin is provided in a reaction buffer solution, followed by addition of the ubiquitination enzymes. In an alternate preferred embodiment, ubiquitin is provided in a reaction buffer solution, a candidate modulator is then added, followed by addition of the ubiquitination enzymes.

In general, a test agent that decreases a POSH ubiquitin-related activity may be used to inhibit POSH function in vivo, while a test agent that increases a POSH ubiquitin-related activity may be used to stimulate POSH function in vivo. A test agent may be modified for use in vivo, e.g., by addition of a hydrophobic moiety, such as an ester.

To perform FP assays, the Ub or Ubl polypeptide is fluorescently labeled. Suitable fluorescent labels are, in view of this specification, well known in the art. Examples are provided below, but suitable fluorescent labels not specifically discussed are also available to those of skill in the art.

Exemplary fluorescent moieties well known in the art include fluorescein, lissamine, phycoerythrin, rhodamine (Perkin Elmer Cetus), Cy2, Cy3, Cy3.5, Cy5, Cy5.5, Cy7, FluorX (Amersham), Texas Red, Alexa Fluor, Oregon Green, indocyanine green, malachite green, BODIPY, dansyl, tetra-methyl rhodamine, and derivatives of fluorescein, benzoxadioazole, coumarin, eosin, Lucifer Yellow, pyridyloxazole and rhodamine. A number of these and many other exemplary fluorescent moieties may be found in the Handbook of Fluorescent Probes and Research Chemicals (2000, Molecular Probes, Inc.), along with methodologies for modifying polypeptides with such moieties.

Exemplary proteins that fluoresce when combined with a fluorescent moiety include, yellow fluorescent protein from *Vibrio fischeri* (Baldwin et al. (1990) Biochemistry 29:5509-15), peridinin-chlorophyll a binding protein from the dinoflagellate *Symbiodinium sp.* (Morris et al. (1994) Plant Molecular Biology 24:673:77) and phycobiliproteins from marine cyanobacteria such as Synechococcus, e.g., phycoerythrin and phycocyanin (Wilbanks et al. (1993) J. Biol. Chem. 268:1226-35). These proteins require flavins, peridinin-chlorophyll a and various phycobilins, respectively, as fluorescent co-factors.

Fluorescent labeling may be accomplished by expressing a polypeptide as a fusion protein with a fluorescent protein, for example fluorescent proteins isolated from jellyfish, corals and other coelenterates. Exemplary fluorescent proteins include the many variants of the green fluorescent protein (GFP) of *Aequoria victoria*. Variants may be brighter, dimmer, or have different excitation and/or emission spectra. Certain variants are altered such that they no longer appear green, and may appear blue, cyan, yellow or red (termed BFP, CFP, YFP and RFP, respectively). Fluorescent proteins may be stably attached to polypeptides through a variety of covalent and noncovalent linkages, including, for example, peptide bonds (e.g., expression as a fusion protein), chemical cross-linking and biotin-streptavidin coupling. For examples of fluorescent proteins, see U.S. Patents 5,625,048; 5,777,079; 6,066,476; 6,124,128; Prasher et al. (1992) Gene, 111: 229-233; Heim et al. (1994) Proc. Natl. Acad Sci., USA, 91:12501-04; Ward et al. (1982) Photochem. Photobiol., 35: 803-808 ; Levine et al. (1982) Comp. Biochem. Physiol., 72B:77-85; Tersikh et al. (2000) Science 290: 1585-88.

The term "output signal" is a general term used to describe any biological event that can be detected in an assay system, such as for example, without limitation; in a transcription-based yeast two hybrid assay, a split ubiquitin assay, etc. A biologically detectable event means an event that changes a measurable property of a biological system, for example, without limitation, light absorbance at a certain wavelength, light emission after stimulation, presence/absence of a certain molecular moiety in the system, electrical resistance/capacitance etc., which event is conditional on another, possibly non-measurable or less easily measurable property of interest of the biological system, for example, without limitation, the presence or absence of an interaction between two proteins. Preferably, the change in the measurable property brought about by the biologically detectable event is large compared to natural variations in the measurable property of the system. Alternatively, other biological functions may be induced and detected following oligomerization, including dimerization, of the output-inducing domains. For example, transcriptional regulation, secondary modification, cell localization, excocytosis, cell signaling, protein degradation or inactivation, cell viability, regulated apoptosis, growth rate, cell size. Such biological events may also be controlled by a variety of direct and indirect means including particular activities associated with individual proteins such as protein kinase or phosphatase activity, reductase activity, cyclooxygenase activity, protease activity or any other enzymatic reaction dependent on subunit association. Also, one may provide for association of G proteins with a receptor protein associated with the cell cycle, e.g., cyclins and cdc kinases, or multiunit detoxifying enzymes.

The use of fluorescent proteins derived from Aequorea victoria has revolutionized research into many cellular and molecular-biological processes. In certain embodiments, the output-inducing peptide comprises a fluorescent protein. The gene sequence encoding a fluorescent protein may be joined in-frame with a gene encoding the protein of interest, e.g., a Ub or Ubl polypeptide, and the desired fusion protein produced when inserted into an appropriate expression vector. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as *E. coli.* A number of vectors exist for the expression of recombinant proteins in yeast. For instance, YEP24, YIP5, YEP51, YEP52, pYES2, and YRP17 are cloning and expression vehicles useful in the introduction of genetic constructs into *S. cerevisiae* (see, for example, Broach et al, (1983) in Experimental Manipulation of Gene Expression, ed. M. Inouye Academic Press, p. 83). Preferred mammalian expression vectors contain both prokaryotic sequences to facilitate the propagation of the vector in bacteria, and one or more eukaryotic transcription units that are expressed in eukaryotic cells. The peDNAI/amp, pcDNAJ/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. The various methods employed in the preparation of the plasmids and transformation of host organisms are well known in the art. For other suitable expression systems for both prokaryotic and eukaryotic cells, as well as general recombinant procedures, see Molecular Cloning A Laboratory Manual, 2nd Ed., ed. by Sambrook, Fritsch and Maniatis (Cold Spring Harbor Laboratory Press, 1989) Chapters 16 and 17.

For example, polymerase chain reaction or complementary oligonucleotides may be employed to engineer a polynucleotide sequence corresponding to the fluorescent protein, 5' or 3' to the gene sequence corresponding to the protein of interest Alternatively, the same techniques may be used to engineer a polynucleotide sequence corresponding to the fluorescent protein sequence 5' or 3' to the multiple cloning site of an expression vector prior to insertion of a gene sequence encoding the protein of interest. The polynucleotide sequence corresponding to the fluorescent protein sequence may comprise additional nucleotide sequences to include cloning sites, linkers, transcription and translation initiation and/or termination signals, labeling and purification tags.

Several examples of fluorescent proteins are known in the art. A well-known example of a fluorescent protein is the native GFP derived from species of the genus Aequorea, suitably Aequorea victoria. The chromophore in wtGFP (native GFP) from Aequorea victoria is at positions 65-67 of the predicted primary amino acid sequence.

The labeled proteins of the present invention may comprise a wtGFP or a fragment thereof that can generate a detectable fluorescence signal.

U.S. Patent No. 5,491,084 describes the use of GFP as a biological reporter. Early applications of GFP as a biological reporter (Chalfie et al. Science, (1994), 263, 802-5; Chalfie, et al, Photochem. Photobiol., (1995), 62 (4), 651-6) used wild type (native) GFP (wtGFP), but these studies quickly demonstrated two areas of deficiency of wtGFP as a reporter for use in mammalian cells. Consequently, significant effort has been expended to produce variant mutated forms of GFP with properties more suitable for use as an intracellular reporter.

A number of mutated forms of GFP with altered spectral properties have been described. A variant-GFP (Heim et al. (1994) Proc. Natl. Acad. Sci. 91, 12501) contains a Y66H mutation which blue-shifts the excitation and emission spectrum of the protein. W096/27675 describes two variant GFPs, obtained by random mutagenesis and subsequent selection for brightness, which contain the mutations V163A and V163A+S175G, respectively. These variants were shown to produce more efficient expression in plant cells relative to wtGFP and to increase the thermo-tolerance of protein folding. The double mutant V163A+S175G was observed to be brighter than the variant containing the single V 163A mutant alone. This mutant exhibits a blue-shifted excitation peak. U.S. Patent No. 6,172,188 describes variant GFPs wherein the amino acid in position 1 preceding the chromophore has been mutate to provide an increase of fluorescence intensity. Such mutations include F641, F64V, F64A, F64G and F64L, with F64L being the preferred mutation. These mutants result in a substantial increase in the intensity of fluorescence of GFP without shifting the excitation and emission maxima. F64L-GFP has been shown to yield an approximate 6-fold increase in fluorescence at 37°C due to shorter chromophore maturation time.

In addition to the single mutants or randomly derived combinations of mutations described above, a variety of variant-GFPs have been created which contain two or more mutations deliberately selected from those described above and other mutations, and which seek to combine the advantageous properties of the individual mutations to produce a protein with expression and spectral properties which are suited to use as a sensitive biological reporter in mammalian cells. U.S. Patent No. 6,194,548 discloses GFPs with improved fluorescence and folding characteristics at 37°C that contain, at least, the changes F64L and V163A and S175G.

U.S. Patent No. 5,777,079 describes a blue fluorescent protein (BFP) containing F64L, S65T, Y66H and Y145F mutations. This is referred to as BFP, because it emits blue fluorescence by UV excitation (R. Heim et al. Curr. Biol. (1996), 6,178-182 ; R. Heim et al. Proc. Natl. Acad. Sci. USA, (1994), 91,12501-12504). However, this BFP was very dim and it experienced severe photo-bleaching as compared to green fluorescent protein. U.S. Patent No. 6,194,548 describes a further BFP containing the F64L, Y66H, Y145F and L236R substitutions. This patent also discloses a mutant containing: F64L, Y66H, Y145F, V163A, S175G, and L236R. Further mutants are described comprising the Y66H, Y145F, V163A and S 175G mutations ; and the F64L, Y66H, and Y145F mutations. Further optional mutations are described at S65T and Y231 L. These mutants are more photostable than those described in U.S. Patent No. 5,777, 079.

WO03029286 describes novel engineered derivatives of blue fluorescent protein (BFP) and nucleic acids that encode engineered BFPs which exhibit more stable fluorescence properties and have different excitation spectra and/or emission spectra relative to wtGFP when expressed in non-homologous cells at temperatures above 30°C, and when excited at about 390 nm. In particular, W003029286 provides novel fluorescent proteins that fluoresce in the blue region of the spectrum ("BFPs'') and have a cellular fluorescence that is more stable than that of BFPs previously described.

WO03062270 describes a colorless protein, acGFP, from Aequorea coerulescens, or fluorescent and non-fluorescent mutants or derivatives of acGFP, as well as fragments and homologs of the nucleic acid compositions. The phrase "fluorescent protein" means a protein that is fluorescent, e.g., it may exhibit low, medium or intense fluorescence upon irradiation with light of the appropriate excitation wavelength. The proteins disclosed in WO03062270 are those in which the fluorescent characteristic is one that arises from the interaction of two or more amino acid residues of the protein, and not from a single amino acid residue. As such, the fluorescent proteins of WO03062270 do not include proteins that exhibit fluorescence only from residues that act by themselves as intrinsic fluors, i.e., tryptophan, tyrosine and phenylalanine. Instead, the fluorescent proteins of W003062270 are fluorescent proteins whose fluorescence arises from some structure in the protein other than the above-specified single amino acid resides; e.g., it arises from an interaction of two or more amino acid residues.

Accordingly, fusion proteins of the present invention may comprise a BFP, selected from the variants described above.

Fusion proteins of the present invention may comprise for example, an acGFP or mutant acGFP polypeptide, as described in WO03/062270 and a second polypeptide (a Ub or Ubl or a prey peptide) fused in-frame at the N-terminus and/or C- terminus of the acGFP polypeptide.

A variety of other reagents may be included in the screening assay. These include reagents like salts, neutral proteins, e.g. albumin, detergents, etc that are used to facilitate optimal protein-protein binding and/or reduce nonspecific or background interactions. Reagents that improve the efficiency of the assay, such as protease inhibitors, nuclease inhibitors, antimicrobial agents, etc. may be used. The mixture of components are added in any order that provides for the requisite binding. Incubations are performed at any suitable temperature, typically between 4° and 40° C. Incubation periods are selected for optimum activity, but may also be optimized to facilitate rapid high-throughput screening.

The application will be more readily understood by reference to the following examples, which are included merely for purposes of illustration of certain aspects and embodiments of the present application, and are not intended to limit the application.

EXEMPLIFICATION

EXAMPLE I

1) Enzymatic step: fluorescent labeled ubiquitin (with or with out cold ubiquitin) are incubated in reaction buffer with E1, E2 and E3 for 30 minutes at 37°C.

2) Formation of poly-ubiquitin chains

3) Readout step: reaction is read in a FP analyst

4) polarization calculation (mP)

**Materials:**

A.) ubiquitin (1-5 ng) (Sigma);

B.) fluorescent labeled ubiquitin (0.1-5 ng) (Boston biochem or in-house production)

C.) Reaction buffer (40 mM Hepes-NaOH, pH 7.5, 1 mM DTT, 2 mM ATP, 5 mM MgCl2),

D.) Recombinant E1 (4-10 ng),

E.) E2 (1-50 ng),

F.) E3 (1-100ng) for 30-60 minutes at 37oC.

G.) 0.5 M EDTA

**Procedure:**

1) fluorescent labeled ubiquitin, with or without cold ubiquitin, are incubated in reaction buffer with E1, E2 and E3 for 30-60 minutes at 37°C.
2) Reactions were stopped with 0.5 M EDTA.
3) Readout in FP analyzer.

**Monoubiquitination formation assay:**

**STEPS**

5) Enzymatic step: fluorescent labeled ubiquitin is incubated in reaction buffer with E1, E2 and E3 for 30 minutes at 37oC.
6) Formation of mono ubiquitin E3
7) Readout step: reaction is read in a FP analyst
8) polarization calculation (mP)

**Materials:**

H.) fluorescent labeled ubiquitin (0.1-5. ng) (Boston biochem, in-house production)

I.) Reaction buffer (40 mM Hepes-NaOH, pH 7.5, 1 mM DTT, 2 mM ATP, 5 mM MgCl2),

J.) Recombinant E1 (4-10 ng),

K.) E2 (1-50 ng),

L.) E3 (1-100ng) for 30-60 minutes at 37oC.

M.)0.5M EDTA

**Procedure:**

1) fluorescent labeled ubiquitin is incubated in reaction buffer with E1, E2 and E3 for 30-60 minutes at 37oC.

2) Reactions were stopped with 0.5M EDTA.

Readout in FP analyzer

**General remarks for FP assays**

1. fluorescent labeled ubiquitin is mixed in a 96-1512 well plate with a final volume up to 200ul

2. Incubations performed at 37°C for 30-60 minutes

3. The plate is read at excitation and emission filter determined by the flourophore of use. The integration time and Z high will be evaluated experimentally.

4. fp is calculated

Fp (maP)= ((I∥-I∥c)-g(I⊥- I⊥-c))/ ((I∥-I∥)c)+g(I⊥- I∥c))* 1000

I∥ and I⊥ =emission intensities of the tracer with the emission polarization parallel and perpendicular to the excitation polarization

I∥c and I∥c = emission intensities of parallel and perpendicular to the excitation polarization from control of wells containing free control ubiquitin.

g= compensation factor

**EXAMPLE II**

1. <u>Assay employing a fluorescent labeled recognition element:</u>

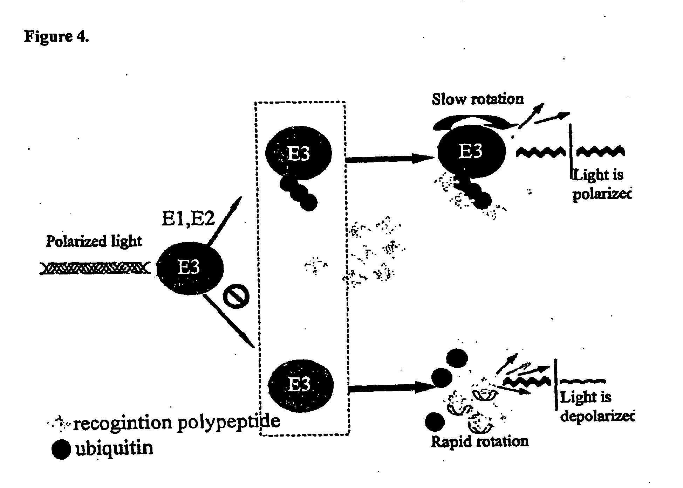

Ubiquitin is initially incubated with E1, E2 and a target E3. After ubiquitin is incorporated randomly into growing polyubiquitin chains, the fluorescently labeled recognition element is added. Chains that contain attached fluorescently labeled peptide are formed and are subsequently measured by FP. (See Fig.4).

Example of polyubiquitination chain recognition assay using fluorescence polarization:

9) Enzymatic step- ubiquitin is incubated in reaction buffer with E1, E2 and E3 for 30-60 minutes at 37°C.

10) Formation of poly-ubiquitin chains

11) Addition of Recognition element.

12) Detection step- depending on type of tag.

<u>**Materials:**</u>

N.) tagged ubiquitin (1-10) nM) (will be added when using TRET/FRET assays);

O.) ubiquitin (1-100nM)

P.) Reaction buffer (40 mM TRIS-Cl/Hepes-NaOH, pH 7.5, 1 mM DTT, 2 mM ATP, 5 mM $MgCl_2$),

Q.) Recombinant E1 (5-50 nM),

R.) $E_2$ (25-200 nM,

S.) E3 (1-10nM),

T.) Recognition element (to be determined)

**Procedure:**

1) Ubiquitin is incubated in reaction buffer with E1, E2 and E3 for 30 minutes at 37°C.
2) Reaction element is added.
3) Readout

Examples of recognition element sequences (protein and peptide):

Example of S5a and S5a peptides f
S5A
gi|5292161|rf|NP_902801.1]
proteasome 26S non-ATPase subunit 4 isoform 1 [Homo sapiens]

MVLESTMVCVDNSEYMRNGDFLPTRLQAQQDAVNIVCHSKTRSNPENNVGL

ITLANDCEVLTTLTPDTGRILSKLHTVQPKGKITFCTGIRVAHLALKHRQGKN

HKMRIIAFVGSPVEDNEKDLVKLAKRLKKEKVNVDIINFGEEEVNTEKLTAFV

NTLNGKDGTGSHLVTVPPGPSLADALISSPILAGEGGAMLGLGASDFEFGVDP

SADPELALALRVSMEEQRQRQEEEARRAAAASAAEAGATTGTEDSDDALLK

MTISQQEFGRTGLPDLSSMTEEEQIAYAMQMSLQGAEFGQAESADIDASSAM

DTSEPAKEEDDYDVMQDPEFLQSVLENLPGVDPNNEAIRNAMGSLASQATKD

GKKDKKEEDKK

| Name: | Length | Pos | Seq: |
|-------|--------|-----|------|
| 5a2 | 45 | 263-307 | MTISQQEPGRTOLPDLSS14TEBEQXAYAZdQKSLQGAEFGQAESAI) |
| 5a2b | 31 | 269-299 | EFGRTGLPDLSSMTEEEQIAYA14QXSLQGA33 |

TAB3

>gi|22749541|ref|NP_690000.1|
TAK1-binding protein 3 isoform 1 [Homo sapiens]

MAQSSPQLDIQVLHDLRQRFPEIPEGVVSQCMLQNNNNLEACCRALSQESSK
YLYMEYHSPDDNRMNRNRLLHINLGIHSPSSYHPGDGAQLNGGRTLVHSSSD
GHIDPQHAAGKQLICLVQEPHSAPAVVAATPNYNPFFMNEQNRSAATPPSQPP
QQPSSMQTGMNPSAMQGPSPPPPPPSYMHIPRYSTNPITVTVSQNLPSGQTVP
RALQILPQIPSNLYGSPGSIYIRQTSQSSSGRQTPQSTPWQSSPQGPVPHYSQRP
LPVYPHQQNYQPSQYSPKQQQIPQSAYHSPPPSQCPSPFSSPQHQVQPSQLGHI
FMPPSPSTTPPHPYQQGPPSYQKQGSHSVAYLPYTASSLSKGSMKKIEITVEPS
QRPGTAINRSPSPISNQPSPRNQHSLYTATTPPSSSPSRGISSQPKPPFSVNPVYIT
YTQPTGPSCTPSPSPRVIPNPTTVFKITVGRATTENLLNLVDQEERSAAPEPIQPI
SVIPGSGGEKGSHKYQRSSSSGSDDYAYTQALLLHQRARMERLAKQLKLEKE
ELERLKSEVNGMEHDLMQRRLRRVSCTTAIPTPEEMTRLRSMNRQLQINVDC
TLKEVDLLQSRGNFDPKAMNNFYDNIEPGPVVPPKPSKKDSSDPCTIERKARR

ISVTSKVQADIHDTQAAAADEHRTGSTQSPRTQPRDEDYEGAPWNCDSCTFL
NHPALNRCEQCEMPRYT

| Name: | Length | Seq: |
|-------|--------|------|
| B3 | 45 | GSTQSPRTQPRDEDYEGAPWNCDSCTFLNWALNRCEQCRMPRYT |
| B3a | 37 | QPRDEDYEGAPWNCDSCTFLNHPALNRCEQCEMPRYT |
| B3a | 29 | GAPWNCDSCTFLNPALNRCEQCBMBRYT |

**Examples of plasmids for production of ubiquitin binding fusion proteins:**

    1. Amplify ubiquitin binding region from ubiquitin binding proteins

        a. from TAB2 amplify codons 540-693
        b. from TAB3 amplify codons 554-712
        c. From S5a amplify codons 196-241, 263-307 or 196-307.

    2. Clone in frame with the coding sequence for GST pGEX system (from Amershem Pharmacia), or a poly histidine tag pETsystem (from Novagen) or another tag which allows protein affinity purification.
    3. Introduce plasmid into bacterial cells such as E. coli BL21
    4. Induce protein expression, harvest cells and purify protein.

2. <u>Assay employing fluorescently labeled ubiquitin and fluorescently labeled recognition element:</u>

The recognition element may be a peptide or a protein. Ubiquitin is initially incubated with E1, E2 and a target E3. After ubiquitin is incorporated randomly into growing poly-ubiquitin chains, the fluorescently labeled recognition peptide is added. Chains that contain attached fluorescent ubiquitin and labeled peptide are formed and are subsequently measured by TRET, FRET, or FLINT.

3. Assay employing radioactively labeled recognition peptide:

Ubiquitin is initially incubated with E1, E2 and a tested E3. After ubiquitin is incorporated randomly into growing poly-ubiquitin chains, the labeled recognition peptide is added. Chains that contain attached radioactively labeled peptide are formed and are subsequently measured by SPA.

**EXAMPLE III**

In vitro self-ubiquitination of untagged hPOSH

Self-ubiquitination is determined by homogenous time-resolved fluorescence resonance energy transfer assay (TR-FRET). The conjugation of ubiquitin cryptate to hPOSH and the binding of anti-hPOSH antibody tagged XL665 bring the two fluorophores into close proximity, which allows the FRET reaction to occur. To measure hPOSH ubiquitination activity, hPOSH (3 ng) is incubated in reaction buffer (40 mM Hepes-NaOH, pH 7.5, 1mM DTT, 2 mM ATP, 5 mM $MgCl_2$), with recombinant E1 (4 ng), UbcH5c (10 ng), ubiquitin (1 ng) and ubiquitin-cryptate (2 ng) (CIS bio International) for 30 minutes at 37°C. Reactions were stopped with 0.5M EDTA. Anti-hPOSH-$XL_{665}$ (in house) (50 nM) was then added to the reaction mixture for a further 45 minutes incubation at room temperature. Emission at 620 nm and 665 nm was obtained after excitation at 380 nm in a fluorescence reader (RUBYstar, BMG Labtechnologies). The generation of hPOSH-ubiquitin-cryptate adducts was then determined by calculating the fluorescence resonance energy transfer (FRET=$\Delta$F) using the following formula:

$$\Delta F= [(S_{665}/S_{620} - B_{665}/B_{620})/(C_{665}/C_{620} - B_{665}/B_{620})]$$

where: S= actual fluorescence, B= Fluorescence obtained in parallel incubation without hPOSH, C= Fluorescence obtained.

**EXAMPLE IV**

**Materials:**

U.) Europium cryptate ubiquitin (11 nM)

V.) ubiquitin (11nM)

W.) Reaction buffer (20 mM TRIS-Cl, pH 7.2, 0.1 mM DTT, 2 mM ATP, 5 mM $MgCl_2$),

X.) Recombinant E1 (10 nM),

Y.) E2 (30 nM),

Z.) E3 (8nM) for 30-60 minutes at 37°C.

AA.) Recognition element S5A-GST 10nM)

Poly chain detection is determined in this example by homogenous time-resolved fluorescence resonance energy transfer assay (TR-FRET). The conjugation of ubiquitin cryptate and ubiquitin into poly chain is detected using an S5A tagged to GST and anti-GST XL665. Formation of the polychain and binding of the S5A will bring the two fluorophores (cryptate and XL 665) into close proximity, which allows the TR-FRET reaction to occur. To measure ubiquitination activity, reagents are added as described above for 60 minutes at 37°C. S5a-GST was added to the Reaction mixture. After 30 minutes incubation in room temperature. Anti-GST-$XCL_{665}$ (Cis bio) (20 nM) was then added to the reaction mixture for a further 45 minutes incubation at room temperature. Emission at 620 nm and 665 nm was obtained after excitation at 380 nm in a fluorescence reader (RUBYstar, BMG Labtechnologies). The generation of hPOSH-ubiquitin-cryptate adducts was then determined by calculating the fluorescence resonance energy transfer (FRET=$\Delta$F) using the following formula

$$\text{EQ1} \quad \Delta F = [(S_{665}/S_{620} - B_{665}/B_{620})/(C_{665}/C_{620} - B_{665}/B_{620})]$$

where: S= actual fluorescence, B= Fluorescence obtained in parallel incubation without hPOSH, Control = Fluorescence obtained.

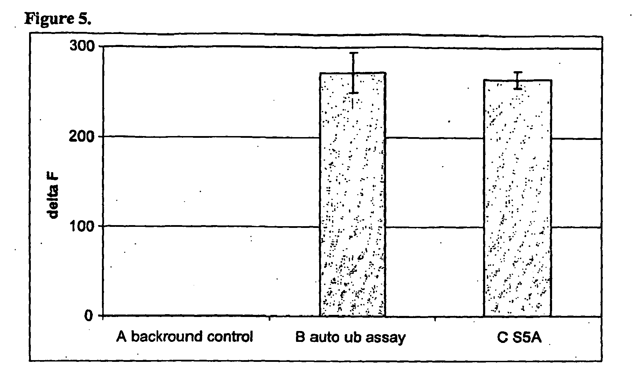

Results are depicted in Figure 5. In Figure 5(A), Background Control is determined as Fluorescence obtained in parallel incubation without E3 (B in Eq1). In Figure 5(B), autoubiqitination is determined by TR-FRET. The conjugation of ubiquitin cryptate to hPOSH and the binding of anti-hPOSH antibody tagged XL665 bring the two fluorophores into close proximity, which allows the FRET reaction to occur. In Figure 5 (C), polychain formation detection by S5a as described above.

**Claims**

1.  A method of evaluating the conjugation of ubiquitin or a ubiquitin-like polypeptide selected from NEDD8, ISG15, SUMO1, SUM02, SUMO3, APG12 or APG8 to a second polypeptide, the method comprising: evaluating the rotational freedom of said ubiquitin or ubiquitin-like polypeptide in a mixture, wherein a decrease in rotational freedom of the ubiquitin or ubiquitin-like polypeptide relative to unconjugated ubiquitin or ubiquitin-like polypeptide indicates that the ubiquitin polypeptide or ubiquitin-like polypeptide is conjugated to a second polypeptide.

2.  The method of claim 1, wherein the ubiquitin or ubiquitin-like polypeptide is labeled with a fluorophore.

3.  The method of claim 2, wherein the rotational freedom of the labeled ubiquitin or ubiquitin-like polypeptide is detected by detecting fluorescence polarization.

4.  The method of any one of claims 1 to 3, wherein evaluating the rotational freedom of ubiquitin or a ubiquitin-like polypeptide in a mixture comprises:

    a) providing a mixture comprising an E3 ubiquitin ligase, and a ubiquitin or ubiquitin-like polypeptide; and
    b) detecting the rotational freedom of the ubiquitin or ubiquitin-like polypeptide.

5.  The method of claims 4, wherein the mixture further comprises a substrate of the E3 ubiquitin ligase.

6.  The method of claim 4 or 5, wherein the mixture further comprises an E1 and an E2.

7.  The method of claim 2 or 3, wherein the fluorophore is selected from: fluorescein or rhodamine.

8.  The method of claim 3, wherein the attachment of more than one ubiquitin or ubiquitin-like polypeptide to the target protein is detected.

9.  A method of identifying a test compound that modulates ubiquitination of a target protein, comprising:

    a) providing a mixture comprising the target protein and a fluorescently labeled ubiquitin or ubiquitin-like polypeptide selected from NEDD8, ISG15, SUMO1, SUMO2, SUMO3, APG12 or APG8; and
    b) detecting the attachment of labeled ubiquitin or ubiquitin-like polypeptide to the protein by fluorescence polarization in the presence and absence of the test compound,

    wherein if the fluorescence polarization of the labeled ubiquitin or ubiquitin-like polypeptide in the presence of the compound is altered relative to the fluorescence polarization of the labeled ubiquitin or ubiquitin-like polypeptide in the absence of the test compound, a test compound that modulates ubiquitination of the protein is identified.

10. The method of any one of claims 3 or 7 to 9, wherein the ubiquitin or ubiquitin-like polypeptide is directly labeled with a fluorophore.

11. The method of any one of claims 3 or 7 to 9, wherein the target protein is an E3.

12. The method of claim 9 or 10, wherein the target protein is selected from the group consisting of: a polypeptide comprising a portion of POSH that is ubiquitinated, a polypeptide comprising a portion of cbl-b that is ubiquitinated, and a polypeptide comprising a portion of PEM-3-like that is ubiquitinated.

13. The method of any one of claims 9 to 12, wherein the mixture further comprises an E2.

14. The method of any one of claims 9 to 13, wherein the mixture further comprises an E1.


**Patentansprüche**

1. Verfahren zur Evaluierung der Konjugation von Ubiquitin oder einem Ubiquitin-ähnlichen Polypeptid ausgewählt aus NEDD8, ISG15, SUMO1, SUMO2, SUMO3, APG12 oder APG8 an ein zweites Polypeptid, wobei das Verfahren umfasst: Evaluieren der Rotationsfreiheit des Ubiquitins oder Ubiquitin-ähnlichen Polypeptids in einer Mischung, wobei eine verringerte Rotationsfreiheit des Ubiquitins oder Ubiquitin-ähnlichen Polypeptids bezogen auf unkonjugiertes Ubiquitin oder Ubiquitin-ähnliches Polypeptid anzeigt, dass das Ubiquitin-Polypeptid oder Ubiquitin-ähnliche Polypeptid an ein zweites Polypeptid konjugiert ist.

2. Verfahren nach Anspruch 1, wobei das Ubiquitin oder Ubiquitin-ähnliche Polypeptid mit einem Fluorophor markiert ist.

3. Verfahren nach Anspruch 2, wobei die Rotationsfreiheit des markierten Ubiquitins oder Ubiquitin-ähnlichen Polypeptids durch den Nachweis von Fluoreszenzpolarisation gemessen wird.

4. Verfahren nach einem der Ansprüche 1 bis 3, wobei die Evaluierung der Rotationsfreiheit des Ubiquitins oder Ubiquitin-ähnlichen Polypeptids in einer Mischung umfasst:

   (a) Bereitstellen einer Mischung umfassend eine E3-Ubiquitin-Ligase und ein Ubiquitin oder Ubiquitin-ähnliches Polypeptid; und
   (b) Messen der Rotationsfreiheit des Ubiquitins oder Ubiquitin-ähnlichen Polypeptids.

5. Verfahren nach Anspruch 4, wobei die Mischung des weiteren ein Substrat für die E3-Ubiquitin-Ligase umfasst.

6. Verfahren nach Anspruch 4 oder 5, wobei die Mischung des weiteren ein E1 und ein E2 umfasst.

7. Verfahren nach Anspruch 2 oder 3, wobei das Fluorophor ausgewählt ist aus: Fluorescein oder Rhodamin.

8. Verfahren nach Anspruch 3, wobei die Bindung von mehr als einem Ubiquitin oder Ubiquitin-ähnlichen Polypeptid an das Zielprotein nachgewiesen wird.

9. Verfahren zur Identifizierung einer Testverbindung, die die Ubiquitinierung eines Zielproteins moduliert, umfassend:

   (a) Bereitstellen einer Mischung umfassend das Zielprotein und ein Fluoreszenz-markiertes Ubiquitin oder Ubiquitin-ähnliches Polypeptid ausgewählt aus NEDD8, ISG15, SUMO1, SUMO2, SUMO3, APG12 oder APG8; und
   (b) Nachweis der Bindung des markierten Ubiquitins oder Ubiquitin-ähnlichen Polypeptids an das Protein mit Hilfe von Fluoreszenzpolarisation in der Gegenwart und Abwesenheit der Testverbindung,

   wobei eine Testverbindung, die die Ubiquitinierung des Proteins moduliert, identifiziert wird, wenn die Fluoreszenzpolarisation des markierten Ubiquitins oder Ubiquitin-ähnlichen Polypeptids in der Gegenwart der Verbindung bezogen auf die Fluoreszenzpolarisation des markierten Ubiquitins oder Ubiquitin-ähnlichen Polypeptids in der Abwesenheit der Testverbindung verändert ist.

10. Verfahren nach einem der Ansprüche 3 oder 7 bis 9, wobei das Ubiquitin oder Ubiquitin-ähnliche Polypeptid direkt mit einem Fluorophor markiert ist.

11. Verfahren nach einem der Ansprüche 3 oder 7 bis 9, wobei das Zielprotein ein E3 ist.

12. Verfahren nach Anspruch 9 oder 10, wobei das Zielprotein ausgewählt ist aus der Gruppe bestehend aus: einem Polypeptid umfassend einen ubiquitinierten Teil von POSH; einem Polypeptid umfassend einen ubiquitinierten Teil von cbl-b und einem Polypeptid umfassend einen ubiquitinierten Teil von einem PEM-3-ähnlichen Protein.

13. Verfahren nach einem der Ansprüche 9 bis 12, wobei die Mischung des weiteren ein E2 umfasst.

14. Verfahren nach einem der Ansprüche 9 bis 13, wobei die Mischung des weiteren ein E1 umfasst.

**Revendications**

1. Procédé d'évaluation de la conjugaison de l'ubiquitine ou d'un polypeptide de type ubiquitine choisi parmi NEDD8, ISG15, SUMO1, SUMO2, SUMO3, APG12 ou APG8 à un second polypeptide, le procédé comprenant : l'évaluation de la liberté de rotation de ladite ubiquitine ou dudit polypeptide de type ubiquitine dans un mélange, où une diminution de la liberté de rotation de l'ubiquitine ou du polypeptide de type ubiquitine par rapport à l'ubiquitine ou au polypeptide de type ubiquitine non conjugué indique que le polypeptide d'ubiquitine ou le polypeptide de type ubiquitine est conjugué à un second polypeptide.

2. Procédé selon la revendication 1, dans lequel l'ubiquitine ou le polypeptide de type ubiquitine est marqué avec un fluorophore.

3. Procédé selon la revendication 2, dans lequel la liberté de rotation de l'ubiquitine ou du polypeptide de type ubiquitine marqué est détectée par la détection de la polarisation de la fluorescence.

4. Procédé selon l'une quelconque des revendications 1 à 3, dans lequel l'évaluation de la liberté de rotation de l'ubiquitine ou d'un polypeptide de type ubiquitine dans un mélange comprend :

   a) la fourniture d'un mélange comprenant une ubiquitine-ligase E3, et une ubiquitine ou un polypeptide de type ubiquitine ; et
   b) la détection de la liberté de rotation de l'ubiquitine ou du polypeptide de type ubiquitine.

5. Procédé selon la revendication 4, dans lequel le mélange comprend en outre un substrat de l'ubiquitine-ligase E3.

6. Procédé selon la revendication 4 ou 5, dans lequel le mélange comprend en outre une E1 et une E2.

7. Procédé selon la revendication 2 ou 3, dans lequel le fluorophore est choisi parmi : la fluorescéine ou la rhodamine.

8. Procédé selon la revendication 3, dans lequel la fixation de plus d'une ubiquitine ou d'un polypeptide de type ubiquitine à la protéine cible est détectée.

9. Procédé d'identification d'un composé à tester qui module l'ubiquitination d'une protéine cible, comprenant :

   a) la fourniture d'un mélange comprenant la protéine cible et une ubiquitine ou un polypeptide de type ubiquitine choisi parmi NEDD8, ISG15, SUMO1, SUMO2, SUM03, APG12 ou APG8, marqué par fluorescence ; et
   b) la détection de la fixation de l'ubiquitine ou du polypeptide de type ubiquitine marqué à la protéine par la polarisation de la fluorescence en présence et en l'absence du composé à tester,

   où si la polarisation de la fluorescence de l'ubiquitine ou du polypeptide de type ubiquitine marqué en présence du composé est modifiée par rapport à la polarisation de la fluorescence de l'ubiquitine ou du polypeptide de type ubiquitine marqué en l'absence du composé à tester, un composé à tester qui module l'ubiquitination de la protéine est identifié.

10. Procédé selon l'une quelconque des revendications 3 ou 7 à 9, dans lequel l'ubiquitine ou le polypeptide de type ubiquitine est marqué directement avec un fluorophore.

11. Procédé selon l'une quelconque des revendications 3 ou 7 à 9, dans lequel la protéine cible est une E3.

12. Procédé selon la revendication 9 ou 10, dans lequel la protéine cible est choisie dans le groupe comprenant : un polypeptide comprenant une partie de POSH qui est ubiquitinée, un polypeptide comprenant une partie de cbl-b qui est ubiquitinée et un polypeptide comprenant une partie de type PEM-3 qui est ubiquitinée.

**13.** Procédé selon l'une quelconque des revendications 9 à 12, dans lequel le mélange comprend en outre une E2.

**14.** Procédé selon l'une quelconque des revendications 9 à 13, dans lequel le mélange comprend en outre une E1.

**Figure 1.**

**E3 bound polyubiquitination chain formation assay.**

**Figure 2.**

**Free polyubiquitination chain formation assay.**

**Figure 3.**

**Mono autoubiquitination formation assay.**

Figure 4.

**Figure 5.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6740495 B
- US 6737244 B
- WO 0050896 A
- US 20020194606 A
- WO 9818956 A
- WO 9805962 A
- US 6326142 B
- US 6100039 A
- US 6202397 B
- US 5786139 A
- WO 9800231 A
- US 20020042083 A
- US 5981200 A
- WO 03095971A2 A
- WO 2002US0036366 A
- WO 03078601A2 A
- WO 2003US0008194 A
- WO 0022110 A
- US 047119 A
- US 5625048 A
- US 5777079 A
- US 6066476 A
- US 6124128 A
- US 5491084 A
- WO 9627675 A
- US 6172188 B
- US 6194548 B
- US 5777 A
- US 079 A
- WO 03029286 A
- WO 03062270 A

### Non-patent literature cited in the description

- **WONG et al.** Drug Discovery in the Ubiquitin Regulatory Pathway. *DDT,* August 2003, vol. 8 (16), 746-54
- **SCHWARTZ ; HOCHSTRASSER.** A Superfamily of Protein Tags: Ubiquitin, SUMO and Related Modifiers. *Trends Biochem. Sci.,* June 2003, vol. 28 (6), 321-28
- **HONG, C. A. et al.** *Assay Drug Dev Technol.,* February 2003, vol. 1 1 (1), 175-80
- **YABUKI, N. et al.** *Comb Chem High Throughput Screen,* October 1999, vol. 2 (5), 279-87
- **LACKOWICZ.** Principles of Fluorescence Spectroscopy. Plenum Press, 1983
- Methods in Fluorescence Polarization
- **NASIR et al.** *Combinatorial Chemistry & High Throughput Screening,* 1999, vol. 2, 177-190
- **LUNDBLAD et al.** *Mol. Endocrinol.,* 1996, vol. 10, 607-612
- **T. M. HSU et al.** *Biotechniques,* 2001, vol. 31, 560-68
- **P. Y. KWOK.** *Hum. Mutat.,* 2002, vol. 19, 315-23
- **HAUGLAND RP.** Handbook of Fluorescent Probes and Research Reagent-Molecular Probes. 2003
- **JANZEN PW.** High through put screening, methods and protocols. Humana press
- **VERVEER et al.** *Science,* 2000, vol. 290, 1567-1570
- **SQUIRE et al.** *J. Microsc.,* 1999, vol. 193, 36
- **VERVEER et al.** *Biophys. J.,* 2000, vol. 78, 2127
- Molecular Probes Catalog. 1996
- Handbook of Fluorescent Probes and Research Chemicals. Molecular Probes, Inc,
- **BALDWIN et al.** *Biochemistry,* 1990, vol. 29, 5509-15
- **MORRIS et al.** *Plant Molecular Biology,* 1994, vol. 24 (673), 77
- **WILBANKS et al.** *J. Biol. Chem.,* 1993, vol. 268, 1226-35
- **PRASHER et al.** *Gene,* 1992, vol. 111, 229-233
- **HEIM et al.** *Proc. Natl. Acad Sci., USA,* 1994, vol. 91, 12501-04
- **WARD et al.** *Photochem. Photobiol.,* 1982, vol. 35, 803-808
- **LEVINE et al.** *Comp. Biochem. Physiol.,* 1982, vol. 72B, 77-85
- **TERSIKH et al.** *Science,* 2000, vol. 290, 1585-88
- **BROACH et al.** Experimental Manipulation of Gene Expression. Academic Press, 1983, 83
- Molecular Cloning A Laboratory Manual. Cold Spring Harbor Laboratory Press, 1989
- **CHALFIE et al.** *Science,* 1994, vol. 263, 802-5

- **CHALFIE et al.** *Photochem. Photobiol.,* 1995, vol. 62 (4), 651-6
- **HEIM et al.** *Proc. Natl. Acad. Sci.,* 1994, vol. 91, 12501
- **R. HEIM et al.** *Curr. Biol.,* 1996, vol. 6, 178-182
- **R. HEIM et al.** *Proc. Natl. Acad. Sci. USA,* 1994, vol. 91, 12501-12504